# EUROPEAN PATENT APPLICATION

(11) **EP 4 067 395 A1**
(43) Date of publication of application: **05.10.2022**
(21) Application number: 20904427.0
(22) Date of filing: 22.12.2020
(51) Int. Cl.: C08F 20/28

(54) **ESTER COMPOUND, METHOD FOR PRODUCING SAME, POLYMER, THERMOSETTING RESIN COMPOSITION AND CURED FILM**

(30) Priority: 27.12.2019 WO PCT/JP2019/051475
(71) Applicant: Kyoeisha Chemical Co., Ltd., Osaka-shi, Osaka 541-0054 (JP)
(72) Inventor: DONKAI, Masaru, Nara-shi, Nara 630-8453 (JP); MORIWAKI, Yuya, Nara-shi, Nara 630-8453 (JP); MATSUDA, Tomoya, Nara-shi, Nara 630-8453 (JP); ASADA, Kosuke, Nara-shi, Nara 630-8453 (JP); NAKAGAWA, Hiroki, Nara-shi, Nara 630-8453 (JP); MAEO, Keiji, Nara-shi, Nara 630-8453 (JP); YOSHIDA, Narutoshi, Nara-shi, Nara 630-8453 (JP); TAKENAKA, Naomi, Nara-shi, Nara 630-8453 (JP)
(74) Representative: Isarpatent
(86) International application number: PCT/JP2020/047986
(87) International publication number: WO 2021/132251

(57) **Abstract**

Provided is a thermosetting resin composition, from which a cured product having excellent transparency with less yellowing than that in the related art can be obtained, and which is advantageous in cost because a monomer obtained by using an inexpensive raw material is used. An ester compound contains, in one molecule, at least one functional group represented by the following general formula (1) or (2) .

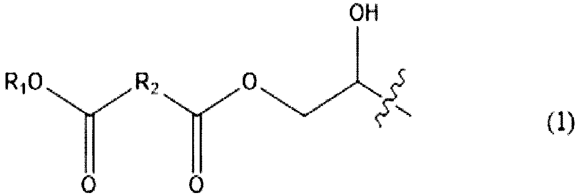

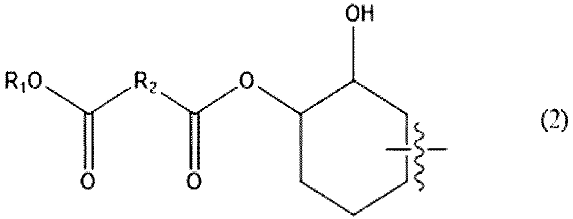

(In both the general formulae (1) and (2), R₁ is an alkyl group having 50 or less carbon atoms.

R₂ is an alkylene group having 50 or less carbon atoms that may contain an oxygen atom and a nitrogen atom as a portion thereof.)

## Description

### Technical Field

The present invention relates to an ester compound, a polymer, a thermosetting resin composition, and a cured film.

### Background Art

The inventors have studied thermosetting resin compositions using a transesterification reaction as a curing reaction (PTL 1 to PTL 6). Through the recent studies, it has become clear that curing performance equivalent to that of curing using generally known melamine resins and polyisocyanate compounds can be secured by using a transesterification reaction as a curing reaction.

Use of such a thermosetting resin composition in various applications has been studied. Therefore, high transparency is also required depending on the application. In the previous thermosetting resin composition in which the transesterification reaction is used as the curing reaction, yellowing may be observed, and improvement may be required for the application in which high transparency is required. Therefore, a method for imparting sufficiently high transparency has been studied. Further, it is also required to use a low-cost raw material produced using an inexpensive material.

### Citation List

### Patent Literature

PTL 1: Japanese Patent No. 6398026
PTL 2: WO 2019/069783
PTL 3: WO 2019/139069
PTL 4: JP-A-2003-119401
PTL 5: WO 2019/203100
PTL 6: WO 2019/240216

### Summary of Invention

### Technical Problem

An object of the invention is to provide a thermosetting resin composition from which a cured product having excellent transparency with less yellowing than that in the related art can be obtained, and which is advantageous in cost because a monomer or a crosslinking agent obtained by using an inexpensive raw material is used.

### Solution to Problem

The invention relates to an ester compound containing, in one molecule, at least one functional group represented by the following general formula (1) or (2).

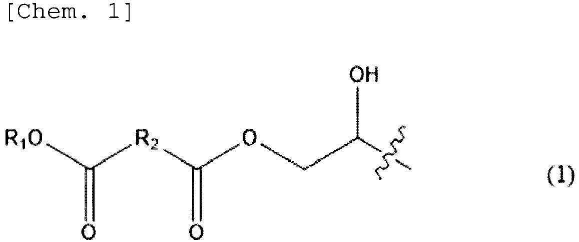

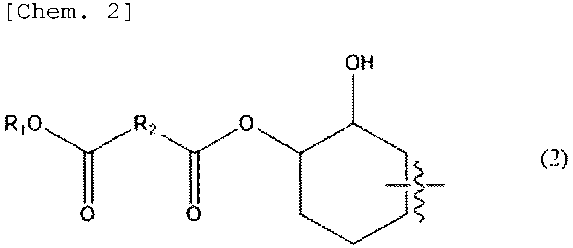

(In both the general formulae (1) and (2), R₁ is an alkyl group having 50 or less carbon atoms.

R₂ is an alkylene group having 50 or less carbon atoms that may contain an oxygen atom and a nitrogen atom as a portion thereof.)

The ester compound may be a compound represented by the following general formula (3).

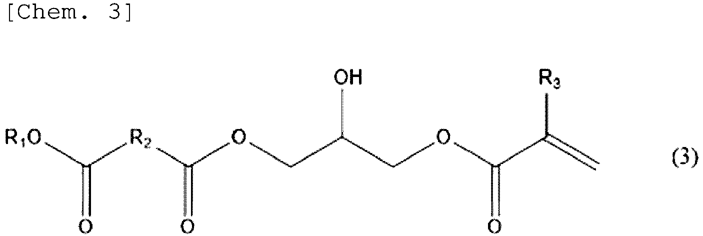

(In the formula, R₁ is an alkyl group having 50 or less carbon atoms.

R₂ is an alkylene group having 50 or less carbon atoms that may contain an oxygen atom and a nitrogen atom as a portion thereof.

R₃ is hydrogen or a methyl group.)

The invention also relates to a method for producing the ester compound, which includes a step of reacting an acid anhydride, an alcohol, and an epoxy compound.

The invention also relates to a polymer containing, as at least a portion thereof, a structural unit derived from the ester compound.

The polymer preferably has a weight average molecular weight of 3,000 to 300,000. The upper limit of the weight average molecular weight is more preferably 100,000, still more preferably 50,000, and even more preferably 30,000. The lower limit of the weight average molecular weight is more preferably 5,000.

The polymer may further contain, as an essential structural unit, a structural unit derived from a hydroxy group-containing unsaturated monomer.

The structural unit derived from a hydroxy group-containing unsaturated monomer contains, as at least a portion thereof, a structural unit derived from a monomer represented by the following general formula (4). 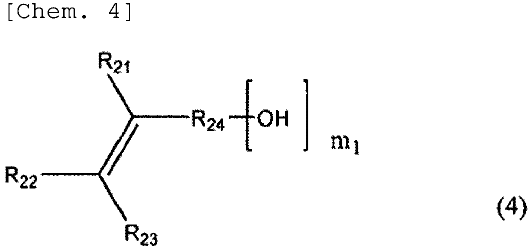 m₁: 1 to 10

(In the formula, R₂₁, R₂₂, and R₂₃ are the same as or different from each other and each represent hydrogen, an alkyl group, a carboxyl group, an alkyl ester group, or a structure represented by the following R₂₄-[OH]m₁.

R₂₄ is an aliphatic, alicyclic, or aromatic alkylene group having 3 or more and 50 or less atoms in a main chain, may contain, in the main chain, one or two or more functional groups selected from the group consisting of an ester group, an ether group, an amide group, and urethane, or may have a side chain.)

The invention also relates to a thermosetting resin composition containing a transesterification catalyst (F) and a resin component (X) containing the functional group and a hydroxy group.

The resin component (X) is preferably any one of the polymers according to the invention.

The invention also relates to a cured film, which is formed by three-dimensionally crosslinking the thermosetting resin composition.

### Advantageous Effect

With the thermosetting resin composition using the novel ester compound according to the invention, a cured product having excellent transparency can be obtained at a low cost.

### Brief Description of Drawings

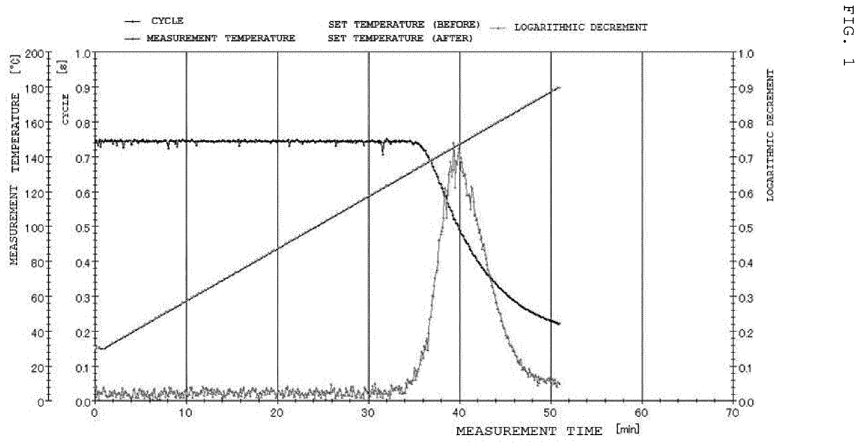

[FIG. 1] FIG. 1 is data of a rigid pendulum tester according to Example 1.

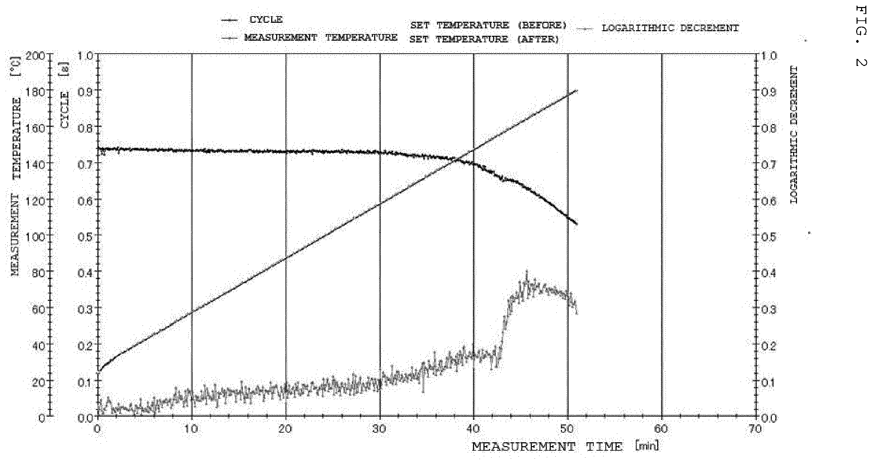

[FIG. 2] FIG. 2 is data of a rigid pendulum tester according to Example 2.

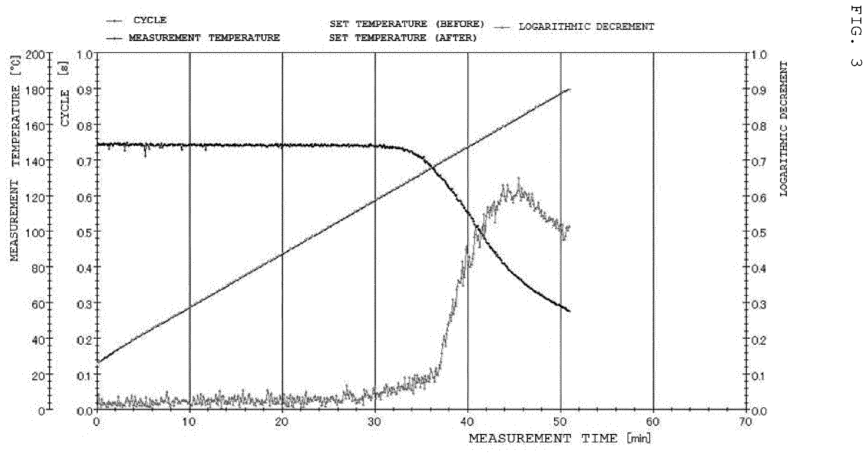

[FIG. 3] FIG. 3 is data of a rigid pendulum tester according to Example 3.

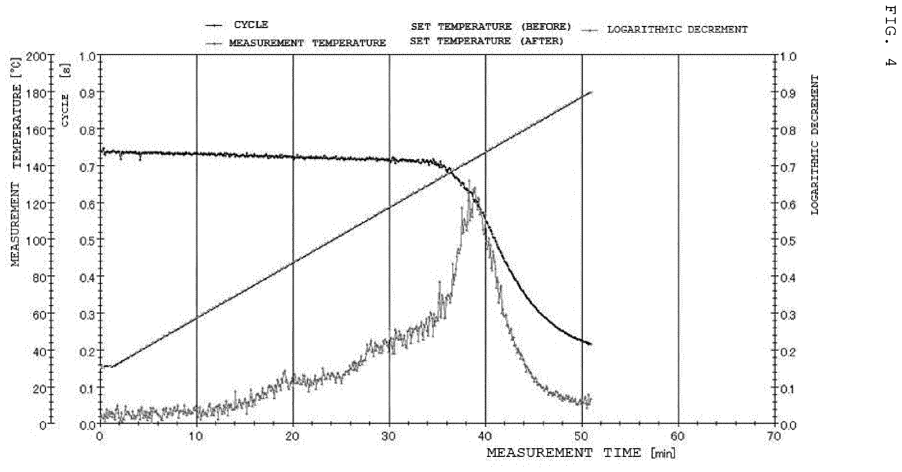

[FIG. 4] FIG. 4 is data of a rigid pendulum tester according to Comparative Example 1.

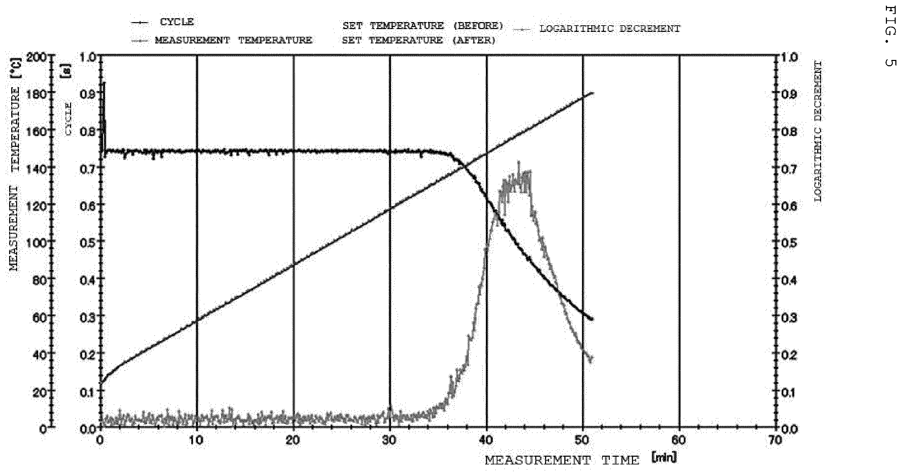

[FIG. 5] FIG. 5 is data of a rigid pendulum tester according to Example 7.

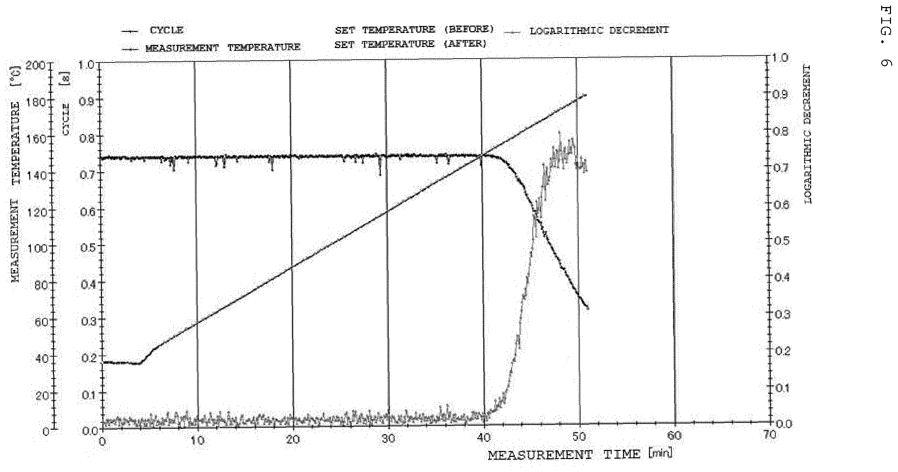

[FIG. 6] FIG. 6 is data of a rigid pendulum tester according to Example 4.

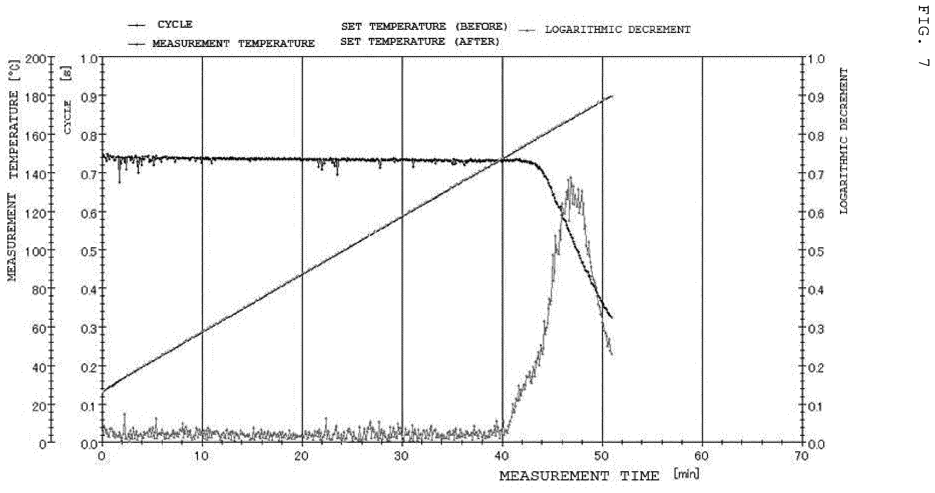

[FIG. 7] FIG. 7 is data of a rigid pendulum tester according to Example 5.

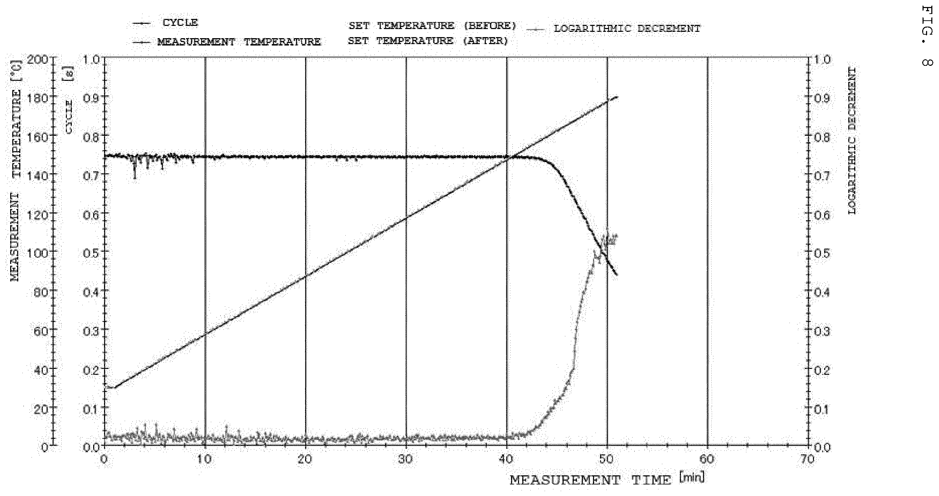

[FIG. 8] FIG. 8 is data of a rigid pendulum tester according to Example 6.

### Description of Embodiments

In a previous thermosetting resin composition in which a transesterification reaction is used as a curing reaction, most of resin components having excellent yellowing resistance and high transparency are costly. An unsaturated group-containing ester compound according to the invention can be produced using a low-cost raw material, and a polymer obtained by polymerization using the unsaturated group-containing ester compound as a monomer is a resin component that does not cause yellowing even after the transesterification reaction and can maintain high transparency.

Further, a crosslinking agent, which is a compound containing two or more functional groups represented by the above-mentioned general formula (1) or (2), also has same excellent performance.

Therefore, according to the invention, it is possible to obtain a thermosetting resin composition in which the above-mentioned problems are solved.

Hereinafter, the invention will be described in detail.

In the following description, "(meth)acrylate" means acrylate and/or methacrylate. "(Meth)acrylic acid" means acrylic acid and/or methacrylic acid. "(Meth)acryloyl" means acryloyl and/or methacryloyl. "(Meth)acrylamide" means acrylamide and/or methacrylamide.

The ester compound according to the invention contains, in one molecule, at least one functional group represented by the following general formula (1) or (2). 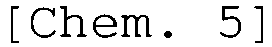 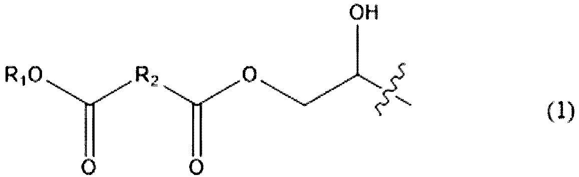 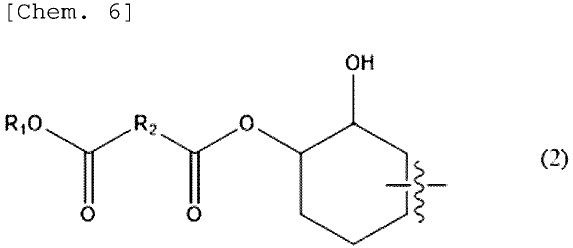 (In both the above-mentioned general formulae (1) and (2), R₁ is an alkyl group having 50 or less carbon atoms.
R₂ is an alkylene group having 50 or less carbon atoms that may contain an oxygen atom and a nitrogen atom as a portion thereof.)

The alkyl group in the alkyl ester group (that is, R₁ in the above-mentioned general formula) is an alkyl group having 50 or less carbon atoms, more preferably an alkyl group having 1 to 20 carbon atoms, still more preferably an alkyl group having 1 to 10 carbon atoms, even more preferably an alkyl group having 1 to 6 carbon atoms, and still even more preferably an alkyl group having 1 to 4 carbon atoms. The alkyl group is most preferably an alkyl group having 1 to 3 carbon atoms. Such a range is preferable in that the curing reaction can suitably proceed.

Specific examples of the alkyl group include those containing a known ester group such as a methyl ester group, an ethyl ester group, a benzyl ester group, an n-propyl ester group, an isopropyl ester group, an n-butyl ester group, an isobutyl ester group, a sec-butyl ester group, and a t-butyl alkyl group. In particular, a methyl group or an ethyl group is preferable, and a methyl group is most preferable. A methyl group is preferable in that reactivity of the thermosetting resin composition is high and a cured resin having excellent xylene rubbing resistance can be obtained.

An R₂ group in the above-mentioned general formula (1) is an alkylene group having 50 or less carbon atoms that may contain an oxygen atom and a nitrogen atom as a portion thereof, and specifically, may include a methylene group, an ethylene group, an n-propylene group, an i-propylene group, an n-butylene group, or a cyclic structure such as a benzene ring or a cyclohexyl ring (carbon chains 1 to 50). Among them, an ethylene group is particularly preferable because the ethylene group is made from an inexpensive raw material and has an excellent reactivity.

Examples of a compound having a structure represented by the above-mentioned general formula (1) may include a compound represented by the following general formula (3).

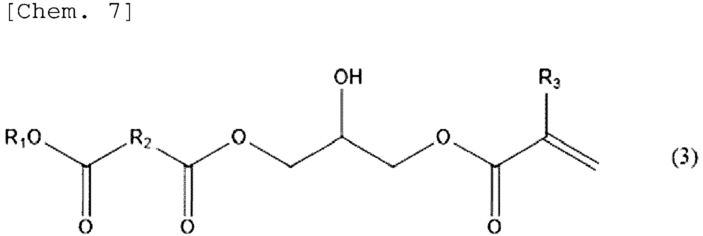

(in the formula, R₁ is an alkyl group having 50 or less carbon atoms.

R₂ is an alkylene group having 50 or less carbon atoms that may contain an oxygen atom and a nitrogen atom as a portion thereof.

R₃ is hydrogen or a methyl group.)

R₂ in the above-mentioned general formula preferably has 50 or less carbon atoms, more preferably 30 or less carbon atoms, still more preferably 20 or less carbon atoms, and even more preferably 10 or less carbon atoms. Further, R₂ is most preferably a hydrocarbon chain, and may be any group having an aromatic ring or an alicyclic structure in the main chain or the side chain.

Among the ester compounds represented by the above-mentioned general formula (3), an ester compound represented by the following general formula (5) is more preferable.

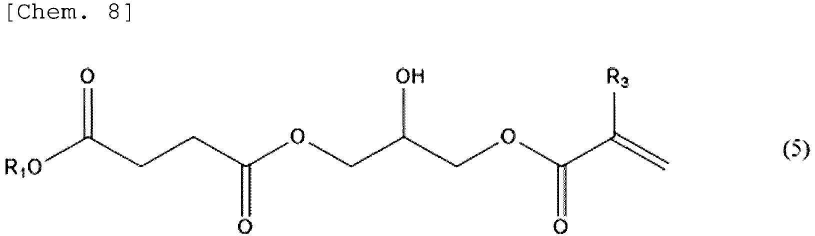

A method of manufacturing an ester compound having a functional group represented by the above-mentioned general formula (1) is not particularly limited, but examples of the method may include a method of reacting an epoxy compound with a compound having an alkyl ester group and a carboxyl group. When this is represented by a general formula, the following reaction is shown.

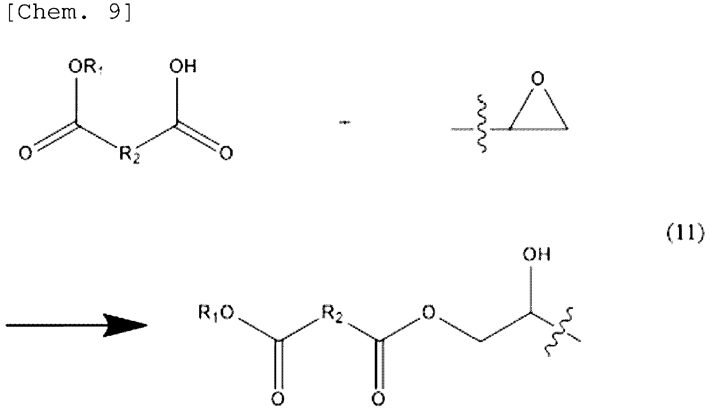

In the above-mentioned reaction, a compound having an alkyl ester group and a carboxyl group to be used can be manufactured by a reaction between acid anhydride and alcohol such as the following reaction.

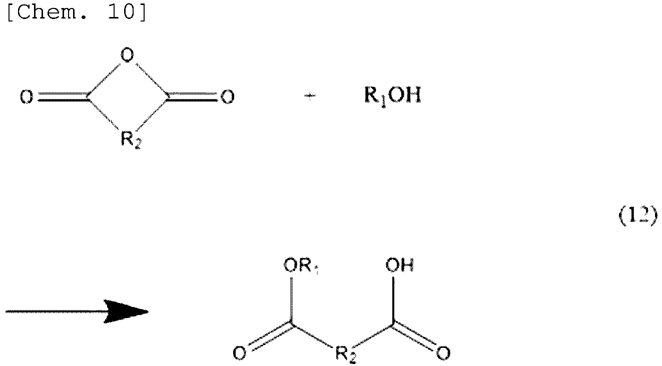

The acid anhydride as a raw material in the reaction represented by the above-mentioned general formula (12) is not particularly limited, and examples thereof include anhydrides of various dibasic acids having a cyclic structure, such as succinic acid anhydride, maleic acid anhydride, phthalic acid anhydride, hexahydrophthalic acid anhydride, methylhexahydrophthalic acid anhydride, tetrahydrophthalic acid anhydride, benzoic acid anhydride, itaconic acid anhydride, citraconic acid anhydride, glutaric acid anhydride, 1,8-naphthalic acid anhydride, 5-norbornene-2,3-dicarboxylic acid anhydride, trimellitic acid anhydride, cantharidin, norcantharidin, adipic acid anhydride, homophthalic acid anhydride, 2,3-pyridinedicarboxylic acid anhydride, hexahydro-4,7-methanoisobenzofuran-1,3-dione, 4-methyl-2,5-oxazolidinedione, 3,4-thiophenedicarboxylic acid anhydride, bicyclo[2.2.2]oct-2-ene-2,3-dicarboxylic acid anhydride, diphenic acid anhydride, diglycolic acid anhydride, het acid anhydride, and isatoic acid anhydride. These acid anhydrides may contain a substituent. The reaction represented by the above-mentioned general formula (12) is a well-known general reaction, and can be performed under general conditions.

Meanwhile, a compound having an alkyl ester group and a carboxyl group which is used in a synthesis method represented by the above-mentioned general formula (11) is not limited to a compound obtained by a method of the above-mentioned general formula (12), and compounds obtained by other methods may also be used.

In the synthesis method represented by the above-mentioned general formula (11), an epoxy compound is used as an essential component. The above-mentioned epoxy compound is not particularly limited as long as it has an unsaturated double bond and an epoxy group, and any epoxy compound can be used.

The epoxy compound that can be used in the above-mentioned reaction may be any known epoxy compound. Examples thereof include glycidyl methacrylate, 4-hydroxybutyl acrylate glycidyl ether, trimethylolpropane triglycidyl ether, an aliphatic polyfunctional liquid epoxy resin, a bisphenol A type epoxy resin, a bisphenol F type epoxy resin, a biphenyl type epoxy resin, a phenol novolac type epoxy resin, a cresol novolac type epoxy resin, a bisphenol derivative epoxy resin, and a novolac type epoxy resin containing a naphthalene skeleton or an alicyclic skeleton, and include an epoxy resin in which an oxirane ring is glycidyl ether.

For example, when epichlorohydrin is used, an epoxy group can be introduced into a compound having various skeletons by reacting the epichlorohydrin with a phenol compound, a carboxylic acid compound, a hydroxyl group-containing compound, or the like. A compound having a functional group represented by the above-mentioned general formula (1) can be obtained by performing the above-mentioned reaction on such any epoxy compound. A general formula of such a reaction is shown below.

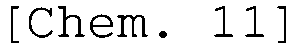

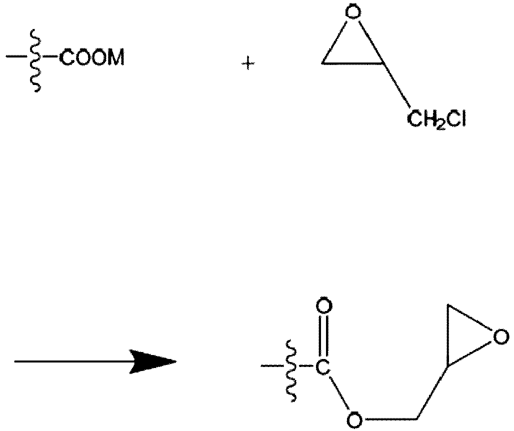

An extremely large number of types of carboxylic acids are known, and various epoxy group-containing compounds can be obtained by performing the above reaction on such a known carboxylic acid. Examples of the known carboxylic acid include polycarboxylic acids containing two or more carboxyl groups, hydroxy carboxylic acids containing a carboxyl group and a hydroxy group, and unsaturated carboxylic acids containing an unsaturated group and a carboxyl group.

The compound represented by the above-mentioned general formula (1) is used as a curable functional group in the curable resin composition. Therefore, a compound containing two or more functional groups is preferable. More specifically, the compound may be a compound containing two or more functional groups represented by the above-mentioned general formula (1), or may be a compound further containing an unsaturated double bond or a hydroxy group in addition to the functional group represented by the above-mentioned general formula (1).

The polycarboxylic acid used here is not particularly limited, and for example, a polycarboxylic acid having 50 or less carbon atoms can be used.

More specifically, examples thereof include: aliphatic polycarboxylic acids such as malonic acid, succinic acid, glutaric acid, adipic acid, pimelic acid, suberic acid, azelaic acid, sebacic acid, undecanedioic acid, dodecanedioic acid, brassylic acid, octadecanedioic acid, citric acid, and butanetetracarboxylic acid;
alicyclic polycarboxylic acids such as 1,2-cyclohexanedicarboxylic acid, 1,3-cyclohexanedicarboxylic acid, 1,4-cyclohexanedicarboxylic acid, 4-cyclohexene-1,2-dicarboxylic acid, 3-methyl-1,2-cyclohexanedicarboxylic acid, 4-methyl-1,2-cyclohexanedicarboxylic acid, 1,2,4-cyclohexanetricarboxylic acid, and 1,3,5-cyclohexanetricarboxylic acid;
aromatic polycarboxylic acids such as phthalic acid, isophthalic acid, terephthalic acid, naphthalenedicarboxylic acid, 4,4'-biphenyldicarboxylic acid, trimellitic acid, and pyromellitic acid;
fatty acids such as coconut oil fatty acids, cottonseed oil fatty acids, hempseed oil fatty acids, rice bran oil fatty acids, fish oil fatty acids, tall oil fatty acids, soybean oil fatty acids, linseed oil fatty acids, tung oil fatty acids, rapeseed oil fatty acids, castor oil fatty acids, dehydrated castor oil fatty acids, and safflower oil fatty acids; and
monocarboxylic acids such as lauric acid, myristic acid, palmitic acid, stearic acid, oleic acid, linoleic acid, linolenic acid, benzoic acid, p-tert-butylbenzoic acid, cyclohexanic acid, and 10-phenyloctadecanoic acid.

Examples of the hydroxy carboxylic acid containing a carboxyl group and a hydroxy group include hydroxy carboxylic acids such as glycolic acid, citric acid, lactic acid, 3-hydroxybutanoic acid, and 3-hydroxy-4-ethoxybenzoic acid.

Examples of the hydroxy carboxylic acid having the above-mentioned carboxyl group and unsaturated group may include (meth)acrylic acid and the like.

Further, the above-mentioned epoxy compound may be a cyclic epoxy compound.

That is, in a case where a cyclic epoxy compound is used as an epoxy compound, a compound having a structure represented by general formula (2) can be obtained by the following reaction.

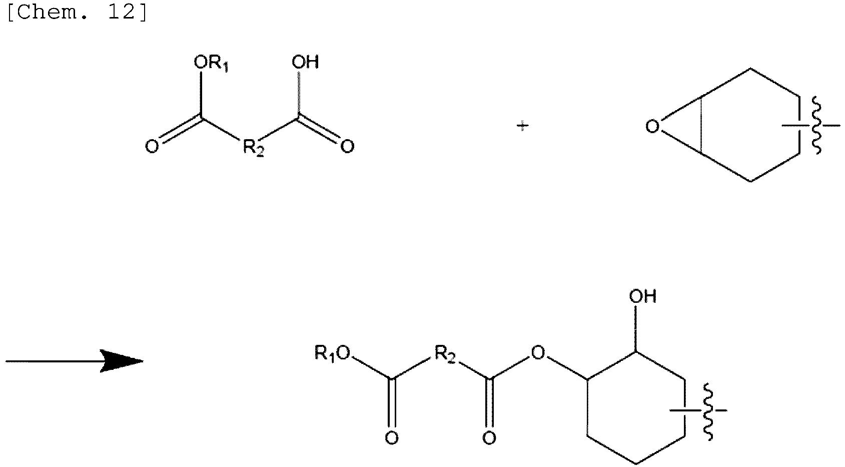

Examples of the alicyclic epoxy compound that can be used in the above-mentioned general formula include 3,4-epoxycyclohexylmethyl methacrylate and 3',4'-epoxycyclohexylmethyl, and 3,4-epoxycyclohexanecarboxylate.

Specific examples of the ester compound according to the invention include compounds represented by the following general formula.

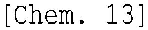

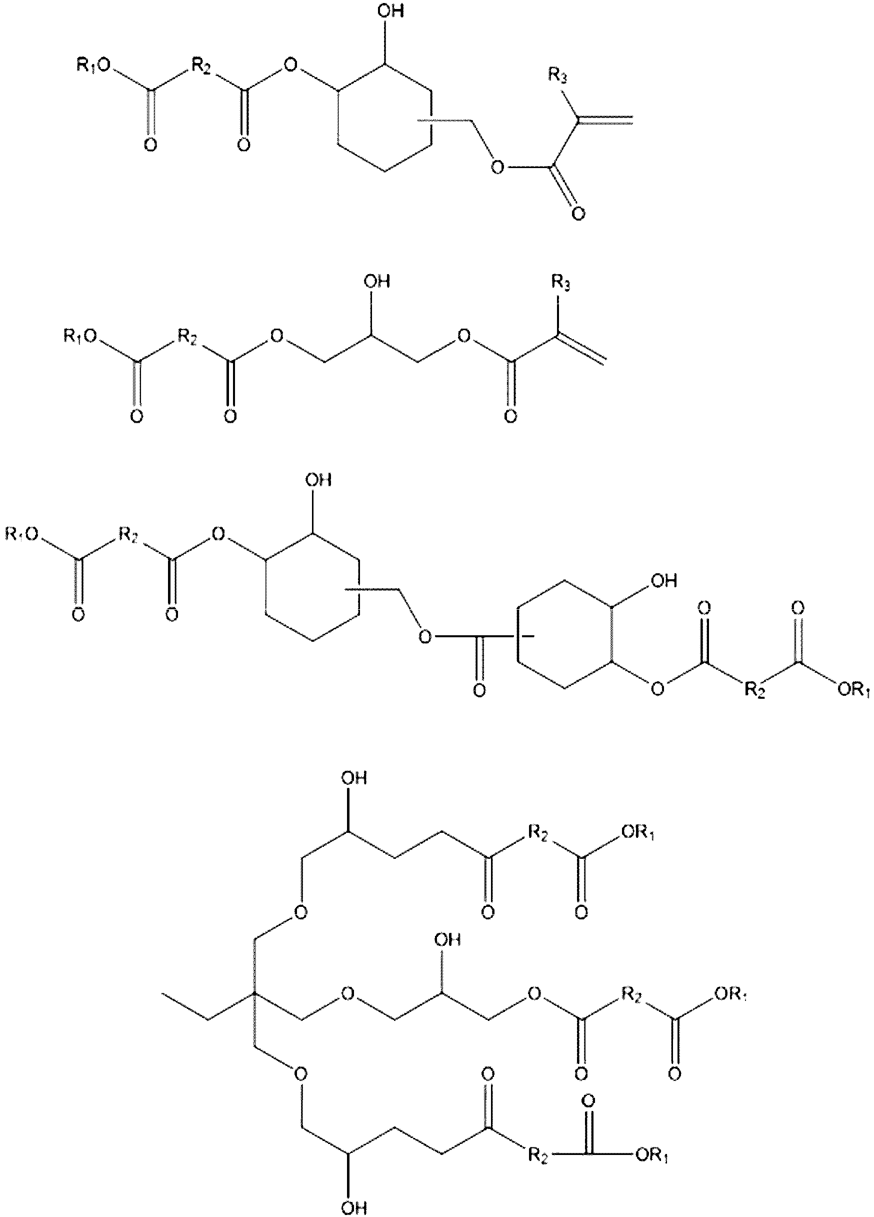

Among the above compounds, in the case of an unsaturated group-containing compound, which is a compound obtained by introducing an epoxy group into a compound containing a carboxyl group, a compound containing an unsaturated group and the functional group represented by the above-mentioned general formula (1) can be obtained.

Such a compound can be used as a polymerizable monomer. Further, a polymer containing the polymerizable monomer as a portion or all thereof can also be used as a component of the thermosetting resin composition in which the transesterification reaction is used as a curing reaction.

This point will be described in detail below.

Further, a compound containing no unsaturated group, which is the above-mentioned compound, can be used as a component of the thermosetting resin composition in combination with other components such as a polyol. The polymer containing the polymerizable monomer as a portion or all thereof and the compound containing no unsaturated group may be used in combination.

### (Polymer)

The polymer according to the invention is a polymer (A) having, as at least a portion thereof, a structural unit derived from the ester compound. That is, the polymer is a homopolymer of the ester compound according to the invention having an unsaturated bond or a copolymer of the ester compound with other monomers. In addition, the polymer may be a polymer (C) containing, as essential structural units, a structural unit derived from the ester compound and a structural unit derived from a hydroxy group-containing unsaturated monomer.

As described above, the main object of the polymer according to the invention is to be used as a component in a curable composition for performing a curing reaction by a transesterification reaction between an alkyl ester group and a hydroxy group. The polymer contains both an alkyl ester group and a hydroxy group in the same molecule. That is, since the alkyl ester group and the hydroxy group are likely to approach each other, the transesterification reaction is likely to occur in one molecule. Since the polymer (C) contains a structural unit derived from a hydroxy group-containing unsaturated monomer, it is considered that the transesterification reaction in the molecule is likely to occur. Further, a curable composition may be obtained by using a hydroxy group-containing compound in combination. This point will be described later.

Other monomers that can be used when the polymer (A) used in the invention is a copolymer will be described in detail below. The hydroxy group-containing monomer can also be used as a copolymerization component, and the hydroxy group-containing monomer and the polymer (C) using the hydroxy group-containing monomer will be described later.

Various α-olefins such as ethylene, propylene, butadiene or butane-1;
various halogenated olefins except fluoroolefin such as vinyl chloride or vinylidene chloride;
various aromatic vinyl compounds such as styrene, α-methylstyrene or vinyltoluene; various amino group-containing amide unsaturated monomers such as N-dimethylaminoethyl(meth)acrylamide, N-diethylaminoethyl(meth)acrylamide, N-dimethylaminopropyl(meth)acrylamide or N-diethylaminopropyl(meth)acrylamide;
various dialkylaminoalkyl(meth)acrylates such as dimethylaminoethyl(meth)acrylate or diethylaminoethyl(meth)acrylate; various amino group-containing monomers such as tert-butylaminoethyl(meth)acrylate, tert-butylaminopropyl(meth)acrylate, aziridinyl ethyl(meth)acrylate, pyrrolidinylethyl(meth)acrylate or piperidinylethyl(meth)acrylate;
various carboxyl group-containing monomers such as (meth)acrylic acid, crotonic acid, itaconic acid, maleic acid or fumaric acid; various epoxy group-containing monomers such as glycidyl(meth)acrylate, β-methylglycidyl(meth)acrylate or (meth)allyl glycidyl ether; mono- or diesters of various α, β-unsaturated dicarboxylic acids such as maleic acid, fumaric acid or itaconic acid with monohydric alcohols having 1 to 18 carbon atoms;
various hydrolyzable silyl group-containing monomers such as vinyltrimethoxysilane, vinyltriethoxysilane, vinyltripropoxysilane, vinylmethyldiethoxysilane, vinyltris (β-methoxyethoxy) silane, allyltrimethoxysilane, trimethoxysilylethyl vinyl ether, triethoxysilylethyl vinyl ether, methyldimethoxysilyl ethyl vinyl ether, trimethoxysilylpropyl vinyl ether, triethoxysilylpropyl vinyl ether, methyldiethoxysilylpropyl vinyl ether, γ-(meth)acryloyloxypropyltrimethoxysilane, γ-(meth)acryloyloxypropyltriethoxysilane or γ-(meth)acryloyloxypropylmethyldimethoxysilane;
various fluorine-containing α-olefins such as vinyl fluoride, vinylidene fluoride, trifluoroethylene, tetrafluoroethylene, chlorotrifluoroethylene, bromotrifluoroethylene, pentafluoropropylene or hexafluoropropylene; various fluorine atom-containing monomers such as various perfluoroalkyl perfluorovinyl ether or (per) fluoroalkyl vinyl ether (provided that the alkyl group has 1 to 18 carbon atoms) including trifluoro methyl trifluorovinyl ether, pentafluoroethyl trifluorovinyl ether or heptafluoropropyl trifluorovinyl ether;
various alkyl vinyl ethers or substituted alkyl vinyl ethers such as methyl vinyl ether, ethyl vinyl ether, n-propyl vinyl ether, isopropyl vinyl ether, n-butyl vinyl ether, isobutyl vinyl ether, tert-butyl vinyl ether, n-pentyl vinyl ether, n-hexyl vinyl ether, n-octyl vinyl ether, 2-ethylhexyl vinyl ether, chloromethyl vinyl ether, chloroethyl vinyl ether, benzyl vinyl ether or phenylethyl vinyl ether;
various cycloalkyl vinyl ethers such as cyclopentyl vinyl ether, cyclohexyl vinyl ether or methyl cyclohexyl vinyl ether; various aliphatic carboxylic acid vinyls such as vinyl 2,2-dimethyl propanoate, vinyl 2,2-dimethyl butanoate, vinyl 2,2-dimethyl pentanoate, vinyl 2,2-dimethyl hexanoate, vinyl 2-ethyl-2-methyl butanoate, vinyl 2-ethyl-2-methyl pentanoate, vinyl 3-chloro-2,2-dimethyl propanoate and the like, as well as vinyl acetate, vinyl propionate, vinyl butyrate, vinyl isobutyrate, vinyl caproate, vinyl caprylate, vinyl caprate or vinyl laurate, C₉ branched aliphatic carboxylic acid vinyl, C₁₀ branched aliphatic carboxylic acid vinyl, C₁₁ branched aliphatic carboxylic acid vinyl or vinyl stearate; vinyl esters of carboxylic acids having a cyclic structure such as vinyl cyclohexane carboxylate, vinyl methyl cyclohexane carboxylate, vinyl benzoate or vinyl p-tert-butylbenzoate.

The polymer (A) may be obtained by using, as a portion of the monomer, an unsaturated group-containing ester compound other than the ester compound according to the invention represented by the general formula (1). The other unsaturated group-containing ester compound is not particularly limited, and examples thereof include methyl (meth)acrylate, ethyl (meth)acrylate, n-propyl (meth)acrylate, benzyl (meth)acrylate, isopropyl (meth)acrylate, n-butyl (meth)acrylate, isobutyl (meth)acrylate, sec-butyl (meth)acrylate, and t-butyl (meth)acrylate.

In the polymer (A), the above-mentioned various monomers may be combined as necessary and polymerized by a known method to obtain a polymer. The polymer (A) preferably contains, as a structural unit in the polymer, the ester compound according to the invention in an amount of 1 wt% to 100 wt%. The lower limit of the content is more preferably 10 wt%, and still more preferably 20 wt%. Within this range, a thermosetting resin composition having good curability can be obtained.

The method for producing the polymer (A) is not particularly limited, and the polymer (A) can be produced by polymerization according to a known method. More specifically, examples thereof include polymerization methods such as a solution polymerization method in an organic solvent, an emulsion polymerization method in water, a mini-emulsion polymerization method in water, an aqueous solution polymerization method, a suspension polymerization method, a UV curing method, anionic polymerization, and cationic polymerization.

In the invention, the polymer (A) preferably has a weight average molecular weight of 3,000 to 300,000. The upper limit of the weight average molecular weight of the polymer is more preferably 100,000, still more preferably 50,000, and even more preferably 30,000. The lower limit of the weight average molecular weight of the polymer (A) is more preferably 3,000, and still more preferably 5,000. The polymer (C) also preferably has the same weight average molecular weight as that of the polymer (A).

### (Thermosetting resin composition)

The thermosetting resin composition according to the invention contains a transesterification catalyst (F) and a resin component (X) containing the functional group contained in the ester compound and the hydroxy group. Specific examples thereof include a compound containing two or more functional groups represented by the above-mentioned general formula (1) or (2), and a compound containing a functional group represented by the above-mentioned general formula (1) or (2) and a hydroxy group.

Hereinafter, the polymer (C) will be described in detail.

The hydroxy group-containing monomer that can be used in the polymer (C) containing an alkyl ester group and a hydroxy group is not particularly limited, and examples thereof include:
In the production of the acrylic resin having both an ester group and a hydroxyl group, it is preferable to use a hydroxyl group-containing vinyl monomer for introducing the hydroxyl group.
Representative examples of the hydroxyl group-containing vinyl monomers are exemplified below;
   various hydroxyl group-containing vinyl ethers such as 2-hydroxyethyl vinyl ether, 3-hydroxypropyl vinyl ether, 2-hydroxypropyl vinyl ether, 4-hydroxybutyl vinyl ether, 3-hydroxybutyl vinyl ether, 2-hydroxy-2-methylpropyl vinyl ether, 5-hydroxypentyl vinyl ether or 6-hydroxyhexyl vinyl ether;
   addition reaction products of these various vinyl ethers and ε-caprolactone;
   various hydroxyl group-containing allyl ethers such as 2-hydroxyethyl (meth)allyl ether, 3-hydroxypropyl (meth)allyl ether, 2-hydroxypropyl (meth)allyl ether, 4-hydroxybutyl (meth)allyl ether, 3-hydroxybutyl (meth)allyl ether, 2-hydroxy-2-methylpropyl (meth)allyl ether, 5-hydroxypentyl (meth)allyl ether or 6-hydroxyhexyl (meth)allyl ether; addition reaction products of these various allyl ethers and ε-caprolactone;
   various hydroxyl group-containing (meth)acrylates such as 2-hydroxyethyl (meth)acrylate, 2-hydroxypropyl (meth)acrylate, 3-hydroxypropyl (meth)acrylate, 2-hydroxybutyl (meth)acrylate, 3-hydroxybutyl (meth)acrylate, 4-hydroxybutyl (meth)acrylate, polyethylene glycol mono (meth)acrylate, or polypropylene glycol mono (meth)acrylate; and
   main components of addition reaction of these various (meth)acrylates and ε-caprolactone.

The hydroxy group-containing monomer as the monomer does not contain a hydroxy group at a position close to the unsaturated bond (specifically, the number of atoms between the hydroxy group and the unsaturated bond is 2 or less), and a hydroxy group-containing monomer containing a hydroxy group via a linking chain having 3 or more and 50 or less atoms is preferable in that the hydroxy group is likely to move in the resin, and thus a transesterification reaction is likely to occur.

That is, when the hydroxy group-containing monomer contains a hydroxy group via a linking chain having 3 or more and 50 or less atoms, the low-temperature curing performance which has not been achieved so far can be obtained by containing both the hydroxy group-containing monomer and the structure derived from the unsaturated group-containing ester compound according to the invention. From the viewpoint of exerting such an unexpected effect, it is preferable to use the above-mentioned hydroxy group-containing monomer. It is presumed that such an effect can be exerted because both the alkyl ester group and the hydroxy group have a high degree of freedom in the resin, and the transesterification reaction is likely to occur.

More specifically, a hydroxy group-containing monomer having a structure represented by the following general formula (4) is preferably used in a portion or all of the hydroxy groups. 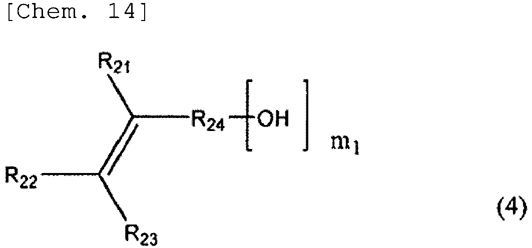 m₁: 1 to 10

(In the formula, R₂₁, R₂₂, and R₂₃ are the same as or different from each other and each represent hydrogen, an alkyl group, a carboxyl group, an alkyl ester group, or a structure represented by the following R₂₄-[OH]m₁.

R₂₄ is an aliphatic, alicyclic, or aromatic alkylene group having 3 or more and 50 or less atoms in a main chain, which may contain, in the main chain, one or more functional groups selected from the group consisting of an ester group, an ether group, an amide group, and urethane, or may have a side chain.)

The compound represented by the above-mentioned general formula (4) is preferably a derivative of (meth)acrylic acid represented by the following general formula (7). 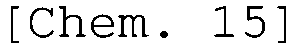 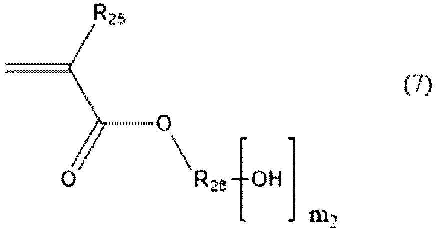 (m₂: 1 to 10

R₂₅ is H or a CH₃.

R₂₆ is an aliphatic, alicyclic, or aromatic alkylene group having 3 or more and 49 or less atoms in a main chain, which may contain, in the main chain, one or two or more functional groups selected from the group consisting of an ester group, an ether group, an amide group, and urethane, or may have a side chain.)

Specific examples of such a compound containing a hydroxy group via a linking chain having, in the main chain, 3 or more and 50 or less molecules include (meth)acrylates such as 3-hydroxypropyl (meth)acrylate, 4-hydroxybutyl (meth)acrylate, 1,4-cyclohexanedimethanol mono(meth)acrylate, polyethylene glycol mono(meth)acrylate, and polypropylene glycol mono (meth) acrylate, and addition reaction main components of the various (meth)acrylates and ε-caprolactone.

The polymer (C) having a structural unit containing the hydroxy group-containing monomer can also be polymerized by the same method as that for the polymer (A).

The thermosetting resin composition according to the invention contains the resin component (X) and the transesterification catalyst (F) according to the present disclosure. Accordingly, a thermosetting resin composition having good curing performance in which the transesterification reaction is used as a curing reaction can be obtained.

The thermosetting resin composition according to the invention may contain the polymer (A), the polymer (C), or both of them. When a polymer containing no hydroxy group is used, the thermosetting resin composition preferably further contains a hydroxy group-containing compound (E). Further, the thermosetting resin composition may be obtained by using a compound containing, in the molecule, at least one functional group represented by the above-mentioned general formula (1) or (2) in combination with the hydroxy group-containing compound (E), instead of a polymer alone or in combination with the polymer (A) or the polymer (C).

When the hydroxy group-containing compound (E) is contained, a transesterification reaction between molecules occurs, and therefore, it is expected that denser three-dimensional crosslinking occurs.

The hydroxy group-containing compound (E) is not particularly limited, and may be a resin or a low molecular weight compound.

Hereinafter, a compound that can be used as the hydroxy group-containing compound (E) will be described in detail.

### Polymer (E-1) of unsaturated monomer containing hydroxy group

Such a polymer can be produced, for example, by copolymerizing a hydroxy group-containing polymerizable unsaturated monomer and another polymerizable unsaturated monomer copolymerizable with the hydroxy group-containing polymerizable unsaturated monomer by a known method. More specifically, examples thereof include polymerization methods such as a solution polymerization method in an organic solvent, an emulsion polymerization method in water, a mini-emulsion polymerization method in water, and an aqueous solution polymerization method.

The hydroxy group-containing polymerizable unsaturated monomer is a compound containing, in one molecule, one or more hydroxy groups and one or more polymerizable unsaturated bonds. As the hydroxy group-containing polymerizable unsaturated monomer, the hydroxy group-containing monomers same as those can be used in the polymer (C) can be used.

The polymer (E-1) of the unsaturated monomer containing a hydroxy group is preferable in that the curing reactivity is good and the low temperature curing reaction can be performed when the monomers represented by the above-mentioned general formulae (4) and (5) are used as a portion or all of the hydroxy group-containing monomer.

Examples of the other polymerizable unsaturated monomer copolymerizable with the hydroxy group-containing monomer include the following monomers (i) to (xiv), and any combination thereof.
(i) Alkyl or cycloalkyl (meth)acrylate:
   methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, n-hexyl (meth)acrylate, n-octyl (meth)acrylate, 2-ethylhexyl (meth)acrylate, nonyl (meth)acrylate, tridecyl (meth)acrylate, lauryl (meth)acrylate, stearyl (meth)acrylate, isostearyl (meth)acrylate, cyclohexyl (meth)acrylate, methylcyclohexyl (meth)acrylate, tert-butylcyclohexyl (meth)acrylate, cyclododecyl (meth)acrylate, tricyclodecanyl (meth)acrylate, etc.
(ii) Polymerizable unsaturated monomer having an isobornyl group:
   isobornyl (meth)acrylate, etc.
(iii) Polymerizable unsaturated monomer having an adamantyl group:
   adamantyl (meth)acrylate, etc.
(iv) Polymerizable unsaturated monomer having a tricyclodecenyl group:
   tricyclodecenyl (meth)acrylate, etc.
(v) Aromatic ring-containing polymerizable unsaturated monomer:
   benzyl (meth)acrylate, styrene, α-methylstyrene, vinyltoluene, etc.
(vi) Polymerizable unsaturated monomer having an alkoxysilyl group:
   vinyltrimethoxysilane, vinyltriethoxysilane, vinyltris(2-methoxyethoxy)silane, γ-(meth)acryloyloxypropyltrimethoxysilane, γ-(meth)acryloyloxypropyltriethoxysilane, etc.
(iv) Polymerizable unsaturated monomer having a tricyclodecenyl group:
   tricyclodecenyl (meth)acrylate, etc.
(v) Aromatic ring-containing polymerizable unsaturated monomer:
   benzyl (meth)acrylate, styrene, α-methylstyrene, vinyltoluene, etc.
(vi) Polymerizable unsaturated monomer having an alkoxysilyl group:
   vinyltrimethoxysilane, vinyltriethoxysilane, vinyltris(2-methoxyethoxy)silane, γ-(meth)acryloyloxypropyltrimethoxysilane, γ-(meth)acryloyloxypropyltriethoxysilane, etc.
(x) Carboxyl group-containing polymerizable unsaturated monomer:
   (meth) acrylic acid, maleic acid, crotonic acid, β-carboxyethyl acrylate, etc.
(xi) Nitrogen-containing polymerizable unsaturated monomer:
   (meth)acrylonitrile, (meth)acrylamide, N, N-dimethylaminoethyl (meth)acrylate, N, N-diethylaminoethyl (meth)acrylate, N, N-dimethylaminopropyl (meth)acrylamide, methylene bis (meth)acrylamide, ethylenebis (meth)acrylamide, adducts of glycidyl (meth)acrylate and an amine compound, etc.
(xii) Polymerizable unsaturated monomer having two or more polymerizable unsaturated groups in one molecule:
   allyl (meth)acrylate, 1,6-hexanediol di(meth)acrylate, etc.
(xiii) Epoxy group-containing polymerizable unsaturated monomer:
   glycidyl (meth)acrylate, β-methylglycidyl (meth)acrylate, 3,4-epoxycyclohexylmethyl (meth)acrylate, 3,4- epoxycyclohexylethyl (meth)acrylate, 3,4-epoxycyclohexylpropyl (meth)acrylate, allyl glycidyl ether, etc.
(xiv) (Meth)acrylate having a polyoxyethylene chain whose molecular terminal is an alkoxy group:
   (xv) Polymerizable unsaturated monomer having a sulfonic acid group:
   2-acrylamido-2-methylpropanesulfonic acid, 2-sulfoethyl (meth)acrylate, allylsulfonic acid, 4-styrenesulfonic acid and the like; sodium salts and ammonium salts etc. of these sulfonic acids.
(xvi) Polymerizable unsaturated monomer having a phosphoric acid group:
   acid phosphoxyethyl (meth)acrylate, acid phosphoxypropyl (meth)acrylate, acid phosphoxy poly(oxyethylene)glycol (meth)acrylate, acid phosphoxy poly(oxypropylene)glycol (meth)acrylate, etc.
(xvii) Polymerizable unsaturated monomer having an ultraviolet absorbing functional group:
   2-hydroxy-4-(3-methacryloyloxy-2-hydroxypropoxy) benzophenone, 2-hydroxy-4-(3-acryloyloxy-2-hydroxypropoxy) benzophenone, 2,2'-dihydroxy-4-(3-methacryloyloxy-2-hydroxypropoxy) benzophenone, 2,2'-dihydroxy-4-(3-acryloyloxy-2-hydroxypropoxy) benzophenone, 2-(2'-hydroxy-5'-methacryloyloxyethylphenyl)-2H-benzotriazole, etc.
(xviii) Ultraviolet stable polymerizable unsaturated monomer:
   4-(meth)acryloyloxy-1,2,2,6,6-pentamethylpiperidine, 4-(meth)acryloyloxy-2,2,6,6-tetramethylpiperidine, 4-cyano-4-(meth)acryloylamino-2,2,6,6-tetramethylpiperidine, 1-(meth)acryloyl-4-(meth)acryloylamino-2,2,6,6-tetramethylpiperidine, 1-(meth)acryloyl-4-cyano-4-(meth)acryloylamino-2,2,6,6-tetramethylpiperidine, 4-crotonoyloxy-2,2,6,6-tetramethylpiperidine, 4-crotonoylamino-2,2,6,6-tetramethylpiperidine, 1-crotonoyl-4-crotonoyloxy-2,2,6,6-tetramethylpiperidine and the like.
(xix) Polymerizable unsaturated monomer having a carbonyl group:
   acrolein, diacetone acrylamide, diacetone methacrylamide, acetoacetoxyethyl methacrylate, formylstyrene, vinyl alkyl ketone having about 4 to about 7 carbon atoms (for example, vinyl methyl ketone, vinyl ethyl ketone, vinyl butyl ketone), and etc.

In the present specification, "polymerizable unsaturated group" means an unsaturated group capable of radical polymerization or ionic polymerization. Examples of the polymerizable unsaturated group include a vinyl group and a (meth)acryloyl group.

A ratio of the hydroxy group-containing monomer in the production of the polymer (E-1) of the unsaturated monomer containing a hydroxy group is preferably 0.5 wt% to 50 wt% based on the total amount of the monomer components. Within such a range, an appropriate crosslinking reaction can be performed, and excellent coating film physical properties can be obtained.

The lower limit is more preferably 1.0 wt%, and still more preferably 1.5 wt%. The upper limit is more preferably 40 wt%.

From the viewpoint of water resistance of the coating film to be formed, the hydroxyl value of the polymer (E-1) of the unsaturated monomer containing a hydroxy group is preferably 1 mgKOH/g to 200 mgKOH/g. The lower limit is more preferably 2 mgKOH/g, and still more preferably 5 mgKOH/g. The upper limit is more preferably 180 mgKOH/g, and still more preferably 170 mgKOH/g.

As such a polymer (E-1) of the unsaturated monomer containing a hydroxy group, a commercially available product can also be used. Commercially available products are not particularly limited, and examples thereof include ACRYDIC A-801-P, A-817, A-837, A-848-RN, A-814, 57-773, A-829, 55-129, 49-394-IM, A-875-55, A-870, A-871, A-859-B, 52-668-BA, WZU-591, WXU-880, BL-616, CL-1000, and CL-408, which are products of DIC Corporation.

In the thermosetting coating material according to the invention, the alkyl ester group can be blended in any ratio with respect to the number of hydroxy groups derived from the polymer (E-1) of the unsaturated monomer containing a hydroxy group. When the ester group is a tertiary ester, the alkyl ester group is preferably 1% to 200% (number ratio).

### (2) Polyester polyol (E-2)

The polyester polyol can usually be produced by an esterification reaction or a transesterification reaction of an acid component and an alcohol component.

As the above-mentioned acid component, a compound which is ordinarily used as an acid component in the production of a polyester resin can be mentioned. Examples of the acid component include aliphatic polybasic acids, alicyclic polybasic acids, aromatic polybasic acids and the like, and anhydrides and esterified products thereof.

As the above-mentioned aliphatic polybasic acid, and anhydride and esterified product thereof, aliphatic compounds having two or more carboxyl groups in one molecule, an acid anhydride of the aliphatic compound and an esterified product of the aliphatic compound are generally mentioned, for example, aliphatic polyvalent carboxylic acids such as succinic acid, glutaric acid, adipic acid, pimelic acid, suberic acid, azelaic acid, sebacic acid, undecane diacid, dodecanedioic acid, brassylic acid, octadecanedioic acid, citric acid, and butanetetracarboxylic acid; anhydrides of the above-mentioned aliphatic polyvalent carboxylic acids; esterified products of lower alkyl having approximately 1 to approximately 4 carbon atoms of the aliphatic polyvalent carboxylic acid, and the like, and any combinations thereof may be mentioned.

The aliphatic polybasic acid is preferably adipic acid and/or adipic anhydride from the viewpoint of the smoothness of the coating film to be obtained.

The above-mentioned alicyclic polybasic acids, and their anhydrides and esterified products are generally compounds having one or more alicyclic structures and two or more carboxyl groups in one molecule, acid anhydrides of the above-mentioned compounds and esterified products of the above-mentioned compounds. The alicyclic structure is mainly a 4- to 6-membered ring structure. Examples of the alicyclic polybasic acid and anhydride and esterified product thereof include the alicyclic polyvalent carboxylic acids such as 1,2-cyclohexanedicarboxylic acid, 1,3-cyclohexanedicarboxylic acid, 1,4-cyclohexanedicarboxylic acid, 4-cyclohexene-1, 2-dicarboxylic acid, 3-methyl-1,2-cyclohexanedicarboxylic acid, 4-methyl-1,2-cyclohexanedicarboxylic acid, 1,2,4-cyclohexanetricarboxylic acid, 1,3,5-cyclohexanetricarboxylic acid and the like; anhydrides of the alicyclic polyvalent carboxylic acids; esterified products of the lower alkyl having approximately 1 to approximately 4 carbon atoms of the alicyclic polyvalent carboxylic acid, and the like; and any combinations thereof may be mentioned.

From the viewpoint of the smoothness of the coating film to be obtained, it is preferable to use 1,2-cyclohexanedicarboxylic acid, 1,2-cyclohexanedicarboxylic anhydride, 1,3-cyclohexanedicarboxylic acid, 1,4-cyclohexanedicarboxylic acid, 4-cyclohexene-1,2-dicarboxylic acid and 4-cyclohexene-1,2-dicarboxylic anhydride, and 1,2-cyclohexane dicarboxylic acid and/or 1,2-cyclohexanedicarboxylic anhydride is more preferable.

The above-mentioned aromatic polybasic acid and their anhydride and esterified product may generally include aromatic polyvalent carboxylic acids such as an aromatic compound having two or more carboxyl groups in one molecule, an acid anhydride of the aromatic compound and an esterified product of the aromatic compound including phthalic acid, isophthalic acid, terephthalic acid, naphthalene dicarboxylic acid, 4,4'-biphenyl dicarboxylic acid, trimellitic acid, pyromellitic acid and the like; acid anhydride of the aromatic polyvalent carboxylic acid, esterified products of lower alkyl having approximately 1 to approximately 4 carbon atoms of the aromatic polyvalent carboxylic acid, and the like, and any combinations thereof.

As the above-mentioned aromatic polybasic acid and their anhydride and esterified product, phthalic acid, phthalic anhydride, isophthalic acid, trimellitic acid, and trimellitic anhydride are preferable.

Further, as the acid component, acid components other than the aliphatic polybasic acid, the alicyclic polybasic acid and the aromatic polybasic acid, for example, fatty acids such as coconut oil fatty acid, cottonseed oil fatty acid, hemp oil fatty acid, rice bran oil fatty acid, fish oil fatty acid, Tall oil fatty acid, soybean oil fatty acid, linseed oil fatty acid, tung oil fatty acid, rapeseed oil fatty acid, castor oil fatty acid, dehydrated castor oil fatty acid, safflower oil fatty acid and the like; monocarboxylic acids such as lauric acid, myristic acid, palmitic acid, stearic acid, oleic acid acid, linoleic acid, linolenic acid, benzoic acid, p-tert-butylbenzoic acid, cyclohexanoic acid, 10-phenyloctadecanoic acid and the like; hydroxy carboxylic acids such as glycolic acid, lactic acid, 3-hydroxybutanoic acid, 3-hydroxy-4-ethoxybenzoic acid, and the like, and any combination thereof may be mentioned.

As the alcohol component, a polyhydric alcohol having two or more hydroxyl groups in one molecule may be used. The polyhydric alcohol may include, for example, dihydric alcohols such as ethylene glycol, propylene glycol, diethylene glycol, trimethylene glycol, tetraethylene glycol, triethylene glycol, dipropylene glycol, 1,4-butanediol, 1,3-butanediol, 2,3-butanediol, 1,2-butane diol, 2-methyl-1,3-propanediol, 3-methyl-1,2-butanediol, 1,1,1-trimethylolpropane, 2-butyl-2-ethyl-1,3-propanediol, 1,2-pentanediol, 1,5-pentanediol, 1,4-pentanediol, 2,4-pentanediol, 2,3-dimethyltrimethylene glycol, tetramethylene glycol, 3-methyl-4,3-pentanediol, 3-methyl-1,5-pentanediol, 2,2,4-trimethyl-1,3-pentanediol, 1,6-hexanediol, 1,5-hexanediol, 1,4-hexanediol, 2,5-hexanediol, neopentyl glycol, 1,4-cyclohexanedimethanol, tricyclodecanedimethanol, hydroxypivalic acid neopentyl glycol ester, hydrogenated bisphenol A, hydrogenated bisphenol F, and dimethylolpropionic acid; polylactone diol obtained by adding a lactone compound such as ε-caprolactone to the dihydric alcohol; ester diol compounds such as bis (hydroxyethyl) terephthalate; polyether diol compounds such as alkylene oxide adducts of bisphenol A, polyethylene glycol, polypropylene glycol and polybutylene glycol; trihydric or higher alcohol such as glycerin, trimethylolethane, trimethylolpropane, diglycerin, triglycerin, 1,2,6-hexanetriol, pentaerythritol, dipentaerythritol, tris (2-hydroxyethyl) isocyanuric acid, sorbitol, and mannitol; a polylactone polyol compound obtained by adding a lactone compound such as ε-caprolactone to the trihydric or higher alcohol; fatty acid esterified products of glycerin, and the like.

As the above-mentioned alcohol component, an alcohol component other than the polyhydric alcohol, for example, a monoalcohol such as methanol, ethanol, propyl alcohol, butyl alcohol, stearyl alcohol or 2-phenoxyethanol; and an alcohol compound obtained by reacting a monoepoxy compound such as propylene oxide, butylene oxide, "Cardura E10" (trade name, glycidyl esters of synthetic hyperbranched saturated fatty acids, manufactured by HEXION Specialty Chemicals, Inc.) with an acid may be used.

The polyester polyol(E-2) is not particularly limited, and it can be produced by a usual method. For example, the acid component and the alcohol component are heated in a nitrogen stream at about 150 to about 250°C. for about 5 to about 10 hours to carry out esterification reaction or transesterification reaction of the acid component and the alcohol component, thereby the polyester polyol can be produced.

### Low molecular weight polyol (E-3)

The above polyol is not limited to the above-mentioned resin, and a low molecular weight polyol (specifically, molecular weight of 2,000 or less) can also be used.

As the low molecular weight polyol, for example, dihydric alcohols such as ethylene glycol, propylene glycol, diethylene glycol, trimethylene glycol, tetraethylene glycol, triethylene glycol, dipropylene glycol, 1,4-butanediol, 1,3-butanediol, 2,3-butanediol, 1,2-butane diol, 2-methyl-1,3-propanediol, 3-methyl-1,2-butanediol, 2-butyl-2-ethyl-1,3-propanediol, 1,2-pentanediol, 1,5-pentanediol, 1,4-pentanediol, 2,4-pentanediol, 2,3-dimethyltrimethylene glycol, tetramethylene glycol, 3-methyl-4,3-pentanediol, 3-methyl-1,5-pentanediol, 2,2,4-trimethyl-1,3-pentanediol, 1,6-hexanediol, 1,5-hexanediol, 1,4-hexanediol, 2,5-hexanediol, neopentyl glycol, 1,4-cyclohexanedimethanol, tricyclodecanedimethanol, hydroxypivalic acid neopentyl glycol ester, hydrogenated bisphenol A, hydrogenated bisphenol F, and dimethylolpropionic acid;
polylactone diol obtained by adding a lactone compound such as ε-caprolactone to the dihydric alcohol;
ester diol compounds such as bis(hydroxyethyl) terephthalate;
polyether diol compounds such as alkylene oxide adducts of bisphenol A, polyethylene glycol, polypropylene glycol and polybutylene glycol;
trihydric or higher alcohol such as glycerin, trimethylolethane, trimethylolpropane, diglycerin, triglycerin, 1,2,6-hexanetriol, pentaerythritol, dipentaerythritol, tris(2-hydroxyethyl) isocyanuric acid, sorbitol, and mannitol.

In the thermosetting resin composition using such a low molecular weight polyol, a low molecular weight polyol, which is a component to be used, is known as a general-purpose product, and is available at a low cost. Further, the low molecular weight polyol has high water solubility, and can be suitably used as a crosslinking agent for the purpose of curing in an aqueous system. In recent years, environmental issues are advocated, and the low molecular weight polyol can be suitably used as a very important crosslinking agent in order to reduce VOC.

As the compound (E) according to the invention, two or more of the polyacrylic polyol (E-1), the polyester polyol (E-2), and the low molecular weight polyol (E-3) may be used in combination.

In the thermosetting coating material according to the invention, the alkyl ester group can be blended in any ratio with respect to the number of hydroxy groups in the entire composition, and when the ester group is a tertiary ester, the alkyl ester group is preferably 1% to 200% (number ratio) with respect to the hydroxy groups.

### Transesterification catalyst (F)

The thermosetting resin composition according to the invention contains the transesterification catalyst (F). That is, the transesterification catalyst (F) is blended in order to efficiently carry out a transesterification reaction between an ester group and a hydroxy group and to obtain sufficient thermosetting performance.

As the transesterification catalyst (F), any known compound capable of activating the transesterification reaction can be used.

Specific examples thereof include various acidic compounds such as hydrochloric acid, sulfuric acid, nitric acid, acetic acid, phosphoric acid, and sulfonic acid; various basic compounds such as LiOH, KOH, NaOH, and amines; and various metal compounds such as PbO, zinc acetate, lead acetate, antimony trioxide, tetraisopropyl titanate, dibutyltin dilaurate, dibutyltin dioctoate, and monobutyltinic acid. In addition, a photoresponsive catalyst or a heat latent catalyst that generates an acid by light or heat can also be used.

Among them, it is desirable to use, as a compound capable of sufficiently exerting the effects of the invention, a compound containing a sulfonic acid group (dodecylbenzenesulfonic acid, phenolsulfonic acid, metasulfonic acid, and p-toluenesulfonic acid) or a compound containing a group including an alkali metal salt or an amine salt of sulfonic acid.

In addition, tin compounds such as dibutyltin dilaurate, dibutyltin dioctoate, dibutyltin oxide, and monobutyltinic acid, aluminum compounds such as aluminum alkylacetoacetate diisopropylate, aluminum monoacetylacetonate bis(ethylacetoacetate), tris(alkylacetoacetate)aluminum, and aluminum acetylacetonate, and metal compounds such as zinc acetylacetonate can also be suitably used as the transesterification catalyst (F).

Since the tin compound and the aluminum compound have high catalytic activity, a thermosetting resin composition having high curing reactivity can be obtained. Further, the resin containing a primary alkyl ester group or a secondary alkyl ester group as the functional group represented by the above-mentioned general formula (1) or (2) is preferable in that the resin can be cured very efficiently.

The transesterification catalyst (F) is preferably used in an amount of 0.01 wt% to 50 wt% based on the total weight of the polymer. Such a range is preferable in that a good curing reaction can be carried out at a low temperature.

The form of the thermosetting resin composition according to the invention is not particularly limited, and it is particularly preferable that the thermosetting resin composition has an organic solvent-based form or an aqueous form. Accordingly, it is preferable in that a thin film can be applied and low-temperature curing can be performed. The aqueous medium may be water-soluble or water-dispersible, and may contain, in addition to water, an aqueous solvent that can be mixed with water at any ratio, such as ethanol, methanol, alcohol-based solvent, a glycol-based solvent, an ether-based solvent, or a ketone-based solvent.

The organic solvent-based thermosetting resin composition is a composition in which the above components are dissolved or dispersed in various organic solvents. The organic solvent that can be used is not particularly limited, and examples thereof include hydrocarbons such as 1-hexane, 1-octane, 1-decane, 1-tetradecane, cyclohexane, benzene and xylene, ethers such as dimethyl ether and diethyl ether, ketones such as acetone, methyl ethyl ketone, and cyclohexanone, chlorinated hydrocarbons such as trichloromethane, carbon tetrachloride, dichloroethane, trichloroethane, tetrachloroethylene and the like, and any known ones such as ethanol, methanol, propanol, butanol, acetone, and the like.

As a two-liquid type resin composition, a solution containing an ester compound and a solution containing a hydroxy group-containing compound may be used in combination by being mixed with each other immediately before use. This is preferable in that the storage stability is improved. Further, a two-liquid type can be also used in which a catalyst solution containing the transesterification catalyst (F) is mixed with a solution containing a composition containing an alkyl ester group and a hydroxy group.

When a thermosetting resin composition having a powder shape such as a powder coating material is used, the thermosetting resin composition can be produced by drying, mixing, and pulverizing the above components by a usual method.

The thermosetting resin composition according to the invention can be suitably used in fields of thermosetting coating materials, thermosetting adhesives, and the like.

When the composition is used as a thermosetting coating material, additives generally used in the field of coating materials may be used in combination with the above-mentioned components. For example, a coloring pigment, an extender pigment, a bright pigment, or any combination thereof may be used in combination.

When a pigment is used, it is preferably contained in a total amount of 1 to 500% by weight, based on 100% by weight of the total solid content of the resin component. The lower limit is more preferably 3% by weight, and still more preferably 5 parts by weight. The upper limit is more preferably 400% by weight, and still more preferably 300% by weight.

Examples of the coloring pigment include titanium oxide, zinc white, carbon black, molybdenum red, prussian blue, cobalt blue, azo pigment, phthalocyanine pigment, quinacridone pigment, isoindoline pigment, threne pigment, perylene pigment, dioxazine type pigment, diketopyrrolopyrrole type pigment, and the like, and any combination thereof.

Examples of the extender pigment include clay, kaolin, barium sulfate, barium carbonate, calcium carbonate, talc, silica, alumina white and the like, and barium sulfate and/or talc is preferable, and barium sulfate is more preferable.

Examples of the bright pigment include, for example, aluminum oxide coated with aluminum (including vapordeposited aluminum), copper, zinc, brass, nickel, aluminum oxide, mica, titanium oxide or iron oxide, mica coated with titanium oxide or iron oxide, glass flakes, hologram pigments, and the like, and any combinations thereof. The aluminum pigment includes nonleafing type aluminum and leafing type aluminum.

The thermosetting coating material may further contain, if desired, a coating material additive such as a thickener, an ultraviolet absorber, a light stabilizer, an antifoaming agent, a plasticizer, an organic solvent other than the hydrophobic solvent, a surface conditioner, and an anti-settling agent.

Examples of the thickener include inorganic thickeners such as silicate, metal silicate, montmorillonite, colloidal alumina and the like; polyacrylic acid thickeners such as copolymers of(meth)acrylic acid and(meth)acrylic acid ester, and sodium polyacrylate; associative type thickener having a hydrophilic part and a hydrophobic part in one molecule and showing a thickening effect by an adsorption of the hydrophobic portion on the surface of the pigment or emulsion particle in the coating material, or an association of the hydrophobic parts, in an aqueous medium; cellulose derivative thickeners such as carboxymethylcellulose, methylcellulose, hydroxyethylcellulose and the like; protein type thickeners such as casein, sodium caseinate, ammonium caseinate and the like; alginic acid thickeners such as sodium alginate; polyvinyl thickeners such as polyvinyl alcohol, polyvinyl pyrrolidone, polyvinyl benzyl ether copolymers; polyether thickeners such as pluronic polyethers, polyether dialkyl esters, polyether dialkyl ethers, polyether epoxy modified products and the like; maleic anhydride copolymer type thickener such as a partial ester of vinyl methyl ether-maleic anhydride copolymer; polyamide type thickeners such as a polyamide amine salt, and the like, and any combination thereof.

The polyacrylic acid thickener is commercially available, and examples thereof include "ACRYSOLASE-60", "ACRYSOLTT-615", and "ACRYSOLRM-5" (trade names) manufactured by Rohm and Haas Company, and "SN Thickener 613", "SN Thickener 618", "SN Thickener 630", "SN Thickener 634", and "SN Thickener 636" (trade names) manufactured by San Nopco CO., LTD.

The associative type thickener is commercially available, and examples thereof include "UH-420", "UH-450", "UH-462", "UH-472", "UH-540", "UH-752", "UH-756VF", and "UH-814N" (trade names) manufactured by ADEKA Corporation, "ACRYSOLRM-8W", "ACRYSOLRM-825", "ACRYSOLRM-2020NPR", "ACRYSOLRM-12W", and "ACRYSOLSCT-275" (trade names) manufactured by Rohm and Haas Company, "SN Thickener 612", "SN Thickener 621 N", "SN Thickener 625 N", "SN Thickener 627 N", and "SN Thickener 660 T" (trade names) manufactured by san nopco CO., LTD. and the like.

The thermosetting resin composition according to the invention may contain at least one curing agent component selected from the group consisting of a polyisocyanate compound, a melamine resin, and an alkoxysilane compound.

Such a curing agent component is not particularly limited, and a polyisocyanate compound, a melamine resin, or an alkoxysilane compound known as a curing agent that reacts with a hydroxy group can be used. More specifically, examples thereof include the following.

### Polyisocyanate compound

The polyisocyanate compound is a compound containing at least two isocyanate groups in one molecule, and examples thereof include aliphatic polyisocyanates, alicyclic polyisocyanates, aromatic aliphatic polyisocyanates, aromatic polyisocyanates, and derivatives of the polyisocyanates.

Examples of the aliphatic polyisocyanate include: aliphatic diisocyanates such as trimethylene diisocyanate, tetramethylene diisocyanate, hexamethylene diisocyanate, pentamethylene diisocyanate, 1,2-propylene diisocyanate, 1,2-butylene diisocyanate, 2,3-butylene diisocyanate, 1,3-butylene diisocyanate, 2,4,4- or 2,2,4-trimethylhexamethylene diisocyanate, dimer acid diisocyanate, and methyl 2,6-diisocyanatohexanoate (common name: lysine diisocyanate); and aliphatic triisocyanates such as 2-isocyanatoethyl 2,6-diisocyanatohexanoate, 1,6-diisocyanato-3-isocyanatomethylhexane, 1,4,8-triisocyanatooctane, 1,6,11-triisocyanatoundecane, 1,8-diisocyanato-4-isocyanatomethyloctane, 1,3,6-triisocyanatohexane, and 2,5,7-trimethyl-1,8-diisocyanato-5-isocyanatomethyloctane.

Examples of the alicyclic polyisocyanate include: alicyclic diisocyanates such as 1,3-cyclopentene diisocyanate, 1,4-cyclohexane diisocyanate, 1,3-cyclohexane diisocyanate, 3-isocyanatomethyl-3,5,5-trimethylcyclohexyl isocyanate (common name: isophorone diisocyanate), 4-methyl-1,3-cyclohexylene diisocyanate (common name: hydrogenated TDI), 2-methyl-1,3-cyclohexylene diisocyanate, 1,3- or 1,4-bis(isocyanatomethyl)cyclohexane (common name: hydrogenated xylylene diisocyanate) or a mixture thereof, methylene bis(4,1-cyclohexanediyl) diisocyanate (common name: hydrogenated MDI), and norbornane diisocyanate; and alicyclic triisocyanates such as 1,3,5-triisocyanatocyclohexane, 1,3,5-trimethylisocyanatocyclohexane, 2-(3-isocyanatopropyl)-2,5-di(isocyanatomethyl)-bicyclo(2.2.1)heptane, 2-(3-isocyanatopropyl)-2,6-di(isocyanatomethyl)-bicyclo(2.2.1)heptane, 3-(3-isocyanatopropyl)-2,5-di(isocyanatomethyl)-bicyclo(2.2.1)heptane, 5-(2-isocyanatoethyl)-2-isocyanatomethyl-3-(3-isocyanatopropyl)-bicyclo(2.2.1)heptane, 6-(2-isocyanatoethyl)-2-isocyanatomethyl-3-(3-isocyanatopropyl)-bicyclo(2.2.1)heptane, 5-(2-isocyanatoethyl)-2-isocyanatomethyl-2-(3-isocyanatopropyl)-bicyclo(2.2.1)heptane, and 6-(2-isocyanatoethyl)-2-isocyanatomethyl-2-(3-isocyanatopropyl)-bicyclo(2.2.1)heptane.

Examples of the aromatic aliphatic polyisocyanate include: aromatic aliphatic diisocyanates such as methylene bis(4,1-phenylene)diisocyanate (common name: MDI), 1,3- or 1,4-xylylene diisocyanate or a mixture thereof, ω,ω'-diisocyanato-1,4-diethylbenzene, and 1,3- or 1,4-bis(1-isocyanato-1-methylethyl)benzene (common name: tetramethylxylylene diisocyanate) or a mixture thereof; and aromatic aliphatic triisocyanates such as 1,3,5-triisocyanatomethylbenzene.

Examples of the aromatic polyisocyanate include: aromatic diisocyanates such as m-phenylene diisocyanate, p-phenylene diisocyanate, 4,4'-diphenyl diisocyanate, 1,5-naphthalene diisocyanate, and 2,4-tolylene diisocyanate (common name: 2,4-TDI) or 2,6-tolylene diisocyanate (common name: 2,6-TDI) or a mixture thereof, 4,4'-toluidine diisocyanate, and 4,4'-diphenyl ether diisocyanate; aromatic triisocyanates such as triphenylmethane-4,4',4"-triisocyanate, 1,3,5-triisocyanatobenzene, and 2,4,6-triisocyanatotoluene; and aromatic tetraisocyanates such as 4,4'-diphenylmethane-2,2' ,5,5'-tetraisocyanate.

Examples of the derivative of the polyisocyanates include dimer, trimer, biuret, allophanate, uretdione, uretonimine, isocyanurate, and oxadiazinetrione of the above-mentioned polyisocyanates, polymethylene polyphenyl polyisocyanate (crude MDI, polymeric MDI), and crude TDI.

As the polyisocyanate compound, the aliphatic diisocyanate, the alicyclic diisocyanate, and the derivatives thereof can be suitably used from the viewpoint of adhesion to the substrate, resistance to cold and heat load, and the like.

As the polyisocyanate compound, a prepolymer obtained by reacting the polyisocyanate and derivatives thereof with a compound capable of reacting with the polyisocyanate under the condition of an excess of isocyanate groups may be used. Examples of the compound capable of reacting with the polyisocyanate include compounds containing an active hydrogen group such as a hydroxy group and an amino group, and specific examples thereof include polyhydric alcohols, low molecular weight polyester resins, amines, and water.

As the polyisocyanate compound, a polymer of an isocyanate group-containing polymerizable unsaturated monomer, or a copolymer of the isocyanate group-containing polymerizable unsaturated monomer with a polymerizable unsaturated monomer other than the isocyanate group-containing polymerizable unsaturated monomer may be used.

Further, the polyisocyanate compound may be a polyisocyanate compound in which an isocyanate group is blocked with a blocking agent, that is, a blocked polyisocyanate compound.

Examples of the blocking agent include: phenols such as phenol, cresol, xylenol, nitrophenol, ethylphenol, hydroxydiphenyl, butylphenol, isopropylphenol, nonylphenol, octylphenol, and methyl hydroxybenzoate; lactams such as ε-caprolactam, δ-valerolactam, γ-butyrolactam, and β-propiolactam; aliphatic alcohols such as methanol, ethanol, propyl alcohol, butyl alcohol, amyl alcohol, and lauryl alcohol; ethers such as ethylene glycol monomethyl ether, ethylene glycol monoethyl ether, ethylene glycol monobutyl ether, diethylene glycol monomethyl ether, diethylene glycol monoethyl ether, propylene glycol monomethyl ether, and methoxymethanol; alcohols such as benzyl alcohol, glycolic acid, methyl glycolate, ethyl glycolate, butyl glycolate, lactic acid, methyl lactate, ethyl lactate, butyl lactate, methylol urea, methylol melamine, diacetone alcohol, 2-hydroxyethyl acrylate, and 2-hydroxyethyl methacrylate; oximes such as formamide oxime, acetoamide oxime, acetooxime, methyl ethyl ketoxime, diacetyl monooxime, benzophenone oxime, and cyclohexane oxime; active methylenes such as dimethyl malonate, diethyl malonate, ethyl acetoacetate, methyl acetoacetate, and acetylacetone; mercaptans such as butyl mercaptan, t-butyl mercaptan, hexyl mercaptan, t-dodecyl mercaptan, 2-mercaptobenzothiazole, thiophenol, methylthiophenol, and ethylthiophenol; acid amides such as acetanilide, acetanisidide, acettoluid, acrylamide, methacrylamide, acetic acid amide, stearic acid amide, and benzamide; imides such as succinimide, phthalimide, and maleic acid imide; amines such as diphenylamine, phenylnaphthylamine, xylidine, N-phenylxylidine, carbazole, aniline, naphthylamine, butylamine, dibutylamine, and butylphenylamine; imidazoles such as imidazole and 2-ethylimidazole; ureas such as urea, thiourea, ethylene urea, ethylene thiourea, and diphenyl urea; carbamic acid esters such as phenyl N-phenylcarbamate; imines such as ethyleneimine and propyleneimine; sulfites such as sodium bisulfite and potassium bisulfite; and azoles. Examples of the azoles include: pyrazoles or pyrazole derivatives such as pyrazole, 3,5-dimethylpyrazole, 3-methylpyrazole, 4-benzyl-3,5-dimethylpyrazole, 4-nitro-3,5-dimethylpyrazole, 4-bromo-3,5-dimethylpyrazole, and 3-methyl-5-phenylpyrazole; imidazoles or imidazole derivatives such as imidazole, benzimidazole, 2-methylimidazole, 2-ethylimidazole, and 2-phenylimidazole; and imidazoline derivatives such as 2-methylimidazoline and 2-phenylimidazoline.

Among them, preferable examples of the blocking agent include an oxime-based blocking agent, an active methylene-based blocking agent, pyrazoles, and a pyrazole derivative.

The blocking (reaction with a blocking agent) can be performed by adding a solvent, if necessary. The solvent used for the blocking reaction is preferably a solvent that is not reactive with the isocyanate group, and examples thereof include ketones such as acetone and methyl ethyl ketone, esters such as ethyl acetate, and solvents such as N-methyl-2-pyrrolidone (NMP).

### Melamine resin

The melamine resin is not particularly limited, and any melamine resin generally used as a curing agent can be used. Alkyl etherified melamine resins are preferable, such as a methyl etherified melamine resin in which a methylol group of a methylolated melamine resin is partially or completely etherified with methyl alcohol, a butyl etherified melamine resin in which a methylol group of a methylolated melamine resin is partially or completely etherified with butyl alcohol, and a methyl-butyl mixed etherified melamine resin in which a methylol group of a methylolated melamine resin is partially or completely etherified with methyl alcohol and butyl alcohol. Such alkyl etherified melamine resins are particularly preferable in terms of excellent curing performance.

### Alkoxysilane compound

The alkoxysilane compound is not particularly limited, and examples thereof include compounds containing a Si-OR group that can be crosslinked by a reaction with a hydroxy group.

More specifically, examples thereof include the following and low molecular weight condensates thereof.

[Chem. 16] Si(Rₐ)ₙ(OR_{b})₄₋ₙ

(Rₐ is an alkyl group having 1 to 50 carbon atoms that may contain a substituent.
R_{b} is an alkyl group having 1 to 4 carbon atoms.
n is an integer of 0 to 2.)

The alkoxysilane compound may be a silicon compound containing, in addition to an alkoxy group, a functional group such as a vinyl group, an epoxy group, an amino group, a (meth)acryloyl group, a carboxyl group, and a mercapto group.

Specific examples of such an alkoxysilane compound include: amino group-containing alkoxysilane compounds such as γ-aminopropyltrimethoxysilane, γ-aminopropylmethyldimethoxysilane, N-(β-aminoethyl)-γ-aminopropyltrimethoxysilane, N-(β-aminoethyl)-γ-aminopropylmethyldimethoxysilane, N-(β-aminoethyl)-γ-aminopropyltriethoxysilane, γ-ureidopropyltriethoxysilane, N-β-(N-vinylbenzylaminoethyl)-γ-aminopropyltrimethoxysilane, and γ-anilinopropyltrimethoxysilane; mercapto group-containing alkoxysilane compounds such as γ-mercaptopropyltrimethoxysilane, γ-mercaptopropyltriethoxysilane, γ-mercaptopropylmethyldimethoxysilane, and γ-mercaptopropylmethyldiethoxysilane; epoxy group-containing alkoxysilane compounds such as γ-glycidoxypropyltrimethoxysilane, γ-glycidoxypropylmethyldimethoxysilane, γ-glycidoxypropyltriethoxysilane, and β-(3,4-epoxycyclohexyl)ethyltrimethoxysilane; carboxy-containing alkoxysilane compounds such as β-carboxyethylphenylbis(2-methoxyethoxy)silane and N-β-(N-carboxymethylaminoethyl)-γ-aminopropyltrimethoxysilane; vinyl group-containing alkoxysilane compounds such as vinyltrimethoxysilane, vinyltriethoxysilane, and vinyltriacetoxysilane; and (meth)acryloyl group-containing alkoxysilane compounds such as 3-methacryloxypropyltrimethoxysilane and 3-methacryloxypropyltriethoxysilane. These compounds may be used alone or in combination of two or more thereof.

Examples of the alkoxysilane compound containing no functional group include a dialkoxysilane, a trialkoxysilane, and a tetraalkoxysilane.

Examples of the dialkoxysilane include dimethoxydimethylsilane, dimethoxydiethylsilane, dimethoxydiphenylsilane, diethoxydimethylsilane, diethoxydiethylsilane, diethoxydiphenylsilane, dipropoxydimethylsilane, dipropoxydiethylsilane, dipropoxydipropylsilane, dipropoxydiphenylsilane, dibutoxydimethylsilane, dibutoxydiethylsilane, dibutoxydibutylsilane, and dibutoxydiphenylsilane.

Examples of the trialkoxysilane include trimethoxymethylsilane, trimethoxyethylsilane, trimethoxypropylsilane, trimethoxybutylsilane, trimethoxyphenylsilane, triethoxymethylsilane, triethoxyethylsilane, triethoxybutylsilane, triethoxyphenylsilane, tripropoxymethylsilane, tripropoxypropylsilane, tripropoxyphenylsilane, and tributoxyphenylsilane.

Examples of the tetraalkoxysilane include tetramethoxysilane, tetraethoxysilane, tetrapropoxysilane, tetrabutoxysilane, and dimethoxydiethoxysilane.

Among the alkoxysilanes, a trialkoxysilane and a tetraalkoxysilane are preferable from the viewpoint of crosslinking performance and the like.

The alkoxy group in these alkoxysilanes is not particularly limited, and examples thereof include an alkoxy group having 1 to 4 carbon atoms, preferably an alkoxy group having 1 to 3 carbon atoms, and more preferably an alkoxy group having 1 to 2 carbon atoms.

The low condensate of at least one of a dialkoxysilane, a trialkoxysilane, and a tetraalkoxysilane may be a low condensate of a monoalkoxysilane, a dialkoxysilane, trialkoxysilane, and a tetraalkoxysilane alone or a low condensate of two or more alkoxysilanes. The low condensate preferably has a degree of polymerization of 10 or less, particularly about 2 to 6. Further, in such a low condensate, a monoalkoxysilane may be used as a portion thereof.

When the curing agent is a polyisocyanate compound and/or a melamine resin, the blending amount (that is, (amount of curing agent)/(amount of curing agent + amount of resin component)) with respect to the total amount of a resin component (B) and the curing agent is preferably 0.01 wt% to 50 wt%. Such a range of the blending amount is preferable in that the curing reaction by the transesterification reaction and the curing reaction by another curing agent are simultaneously carried out.

The lower limit is more preferably 0.01 wt%, and still more preferably 1 wt%. The upper limit is more preferably 30 wt%, and still more preferably 20 wt%.

When the curing agent is an alkoxysilane compound, (amount of curing agent)/(amount of curing agent + amount of resin component) is preferably 0.001 wt% to 10 wt%. Such a range of the blending amount is preferable in that the curing reaction by the transesterification reaction and the curing reaction by another curing agent are simultaneously carried out.

In the invention, sufficient curing performance is exerted even when the blending ratio of the curing agent, that is, (amount of curing agent)/(amount of curing agent + amount of resin component) is a relatively low ratio of 1 wt% to 20 wt%. It is presumed that the reason is that curing by the resin component (B) preferentially proceeds, and the curing reaction by the curing agent supplementarily proceeds, whereby the curing reaction is promoted.

The curing agent component may be a combination of two or more of the polyisocyanate compound, the melamine resin, and the alkoxysilane compound described above.

### (Method for forming multilayer coating film)

The thermosetting resin composition according to the invention can also be used as a coating composition in a method for forming a multilayer coating film, which includes a step of applying a base coating material and a step of applying a clear coating material.

In the method for forming a multilayer coating film, the coating composition according to the invention can be used as one or both of the base coating material and the clear coating composition. The composition of the coating material when used in such applications can be prepared based on the above-mentioned description of the thermosetting resin composition.

In the method for forming a multilayer coating film including the step of applying the base coating material and the step of applying the clear coating material, the coating method is not particularly limited, and a method for forming a multilayer coating film by two-coat one-bake including a step of applying the base coating material, then applying the clear coating material in a wet-on-wet manner, and simultaneously curing the formed uncured multilayer coating film, or a method of forming a multilayer coating film by three-coat one-bake including a step of applying a first base coating material, a second base coating material, and the clear coating material in a wet-on-wet manner, and simultaneously curing the formed uncured multilayer coating film can be used.

Also, a method comprising curing the base coating film after applying the base coating composition, and then curing a base coating film after applying the clear coating composition on the cured coating film.

Although it can be applied to any of the above-mentioned methods, the method of applying the base coating composition and the clear coating composition in a wet-on-wet manner is particularly preferable because it is simple in the process, and is generally used in fields such as automotive coating. In such a method, problems such as a mixed layer between coating films and curing defects due to volatile components are likely to occur. However, when the above-described clear coating composition is used, such a wet-on-wet coating can be performed without any problem.

In the method for forming a multilayer coating film described above, any one of the layers may be coated with an aqueous coating material. When the thermosetting resin composition according to the invention is used as an aqueous coating material, the thermosetting resin composition can be made aqueous by the method described above. In particular, an aqueous base coating material is preferably used as the base coating material. For example, in the method of performing coating by a wet-on-wet manner using an aqueous base coating material and a solvent-based clear coating material, the thermosetting resin composition according to the invention can be used for either or both of the aqueous base coating material and the solvent-based clear coating material.

Hereinafter, a case where the thermosetting resin composition according to the invention is used as an aqueous base coating material will be described in detail. The thermosetting resin composition according to the invention can be made aqueous by the method described above.

In the field of automotive top coating compositions, many metallic coatings in which a bright pigment is blended into a base coating composition are applied.

The bright pigment is not particularly limited, and a bright pigment known in the coatings field can be used. Specific examples include non-colored or colored metallic bright materials such as metals or alloys such as aluminum, aluminum oxide, copper, zinc, iron, nickel and tin. In addition, bright pigments include metal-deposited film flakes, mica, mica surface coated with a metal oxide, mica-like iron oxide, graphite pigments, and light interference pigments including hologram pigments. The shape of the bright pigment is not particularly limited, and may be further colored or may be treated with various surface treating agents, dispersants, and the like. These bright pigments can be used alone or in combination of two or more.

The bright pigment may be appropriately mixed with a dispersant and a dispersing resin, dispersed and formed into a paste, and then blended into a coating composition.

In addition to the bright pigment, the above-mentioned water-borne base coating composition may contain other bright pigments and pigments such as coloring pigments and extender pigments as necessary.

The water-borne base coating composition may further contain, if necessary, other additives for coating composition commonly used in the preparation of water-borne coating compositions, such as an ultraviolet absorber, a light stabilizer, a surface conditioner, polymer fine particles, a pigment dispersant, an anti-settling agent, a thickener, a defoaming agent, a curing catalyst, a deterioration inhibitor, and organic solvents.

Preferably, the water-borne base coating composition generally has a solid content of from 5 to 35% by weight, particularly from 10 to 25% by weight, from the viewpoint of the bright-looking of the formed coating film. Preferably, the water-borne base coating composition usually has a pH in the range of 7.0 to 9.0, particularly 7.5 to 8.5.

Further, two layers of base coating film may be present. In this case, the two layers may have the same resin composition or different resin compositions. Also, only one may be a water-borne coating composition, or both may be water-borne coating compositions. Further, both may be solvent-based coating compositions. However, it is most preferred that both are water-borne base coating compositions.

That is common that these are a first base having an intermediate coating function showing an excellent ultraviolet blocking ability and an excellent adhesion to an electrodeposition coating film surface, and a second base for imparting designability including aluminum, mica, coloring pigment and the like. After performing the first base coating, the second base coating is performed in a wet-on-wet manner, and then a preheating at 50 to 100°C for 1 to 5 minutes is performed as necessary. Further, a clear coating is performed in a wet-on-wet manner. Finally, it is preferable to form a multilayer coating film by a three-coat one-bake process in which three uncured coating films are simultaneously thermally cured.

In the present specification, the solid content of the base coating composition is a weight ratio of a non-volatile content after drying the water-borne base coating composition at 105°C for 1 hour, and can be calculated from the difference between the weights of the coating compositions before drying and after drying. The drying process is performed in the following steps; about 2 g of the bright pigment-containing water-borne base coating composition is measured in an aluminum foil cup of about 5 cm in diameter, spreaded sufficiently on the bottom of the cup, and then dried at 105°C for 1 hour.

The method of applying the bright pigment-containing water-borne base coating composition is not particularly limited, and examples thereof include methods such as air spray coating, electrostatic air spray coating, and electrostatic bell coating. A wet film can be formed on the object to be coated by these methods.

In the case of air spray coating, electrostatic air spray coating or electrostatic bell coating, the viscosity of the water-borne base coating composition is preferably adjusted to a viscosity range suitable for the coating, usually 15 to 60 seconds at 20°C in a Ford cup #4 viscometer, by using water and/or an organic solvent.

Curing of the formed wet coating film can be performed by heating. The heating can be performed by known heating means, for example, using a drying furnace such as a hot blast furnace, an electric furnace, or an infrared induction heating furnace. The heating temperature is usually in the range of about 80 to about 180°C, and preferably about 120 to about 160°C. The heating time is not particularly limited, but can usually be about 10 to 40 minutes.

Suitably, the film thickness of the base coating film is usually in the range of 5 to 20 µm, and preferably 10 to 15 µm as a cured film thickness.

In addition, a multilayer coating film can be formed by a two-coat one-bake method in which a base coating composition is applied on an object to be coated, and a clear coating composition is applied thereon without curing the base coating film, and the base coating film and the clear coating film are simultaneously heated and cured. If the surface to be coated is an uncured intermediate coating film surface, a three-coat one-bake method can be used.

When a multilayer coating film is formed by the two-coat one-bake method, from the viewpoint of preventing the occurrence of coating film defects such as repelling and so on, after the base coating composition is applied, a preheating is preferably performed at a temperature at which the coating film is not substantially cured. The preheating temperature can be generally about 50 to 100°C, and the preheating time can be about 1 to 10 minutes, and preferably about 2 to 5 minutes.

The object to which the thermosetting coating composition can be applied is not particularly limited, and examples thereof include an outer plate portion of an automobile body such as a passenger car, a truck, a motorcycle, and a bus; an automobile part; house electrical products such as a mobile phone, an audio device, etc., building materials, furniture, adhesives, film and glass coating agents, and the like. When used as an automotive coating, it can be used for the curing of an arbitrary layer such as an intermediate coating, a base coating and a clear coating.

The object to be coated may be an object in which a metal material and a metal surface of a vehicle body or the like molded from the metal material is subjected to a surface treatment such as a phosphate treatment, a chromate treatment, or a composite oxide treatment, or may be an object having a coating film.

Examples of the object to be coated having a coating film include an object obtained by subjecting a substrate to a surface treatment as desired and forming an undercoat coating film thereon. In particular, a vehicle body on which an undercoat coating film is formed by an electrodeposition coating material is preferable, and a vehicle body on which an undercoat coating film is formed by a cationic electrodeposition coating material is more preferable.

The object to be coated may be an object in which a plastic material or a plastic surface of an automobile part molded from the plastic material is subjected to a surface treatment, primer coating, or the like, if desired. In addition, the plastic material and the metal material may be combined. The thermosetting resin composition according to the invention can be cured at a low temperature, and thus can be suitably used as a plastic coating material.

The method of applying the thermosetting coating is not particularly limited, and examples thereof include an air spray coating, an airless spray coating, a rotary atomization coating, a curtain coating and the like, and air spray coating, rotary atomization coating, and the like are preferable. At the time of coating, electrostatic application may be performed if desired. By the above-mentioned coating method, a wet coating film can be formed from the water-borne coating material composition.

The wet coating film can be cured by heating. The curing can be carried out by a known heating means, for example, a drying oven such as an air-heating furnace, an electric furnace, an infrared induction heating furnace or the like. The wet coating film is preferably cured by heating at a temperature in the range of approximately 80 to approximately 180°C, more preferably approximately 100 to approximately 170°C, and even more preferably approximately 120 to approximately 160°C, and preferably for approximately 10 to approximately 60 minutes, and more preferably for approximately 15 to approximately 40 minutes. It is also preferable in that it can cope with low temperature curing at 80 to 140°C.

When the thermosetting resin composition according to the invention is used in the field of coating materials, it is required to have sufficient curing performance such as smoothness, water resistance, and acid resistance.

On the other hand, when used in the field of an adhesive, a pressure-sensitive adhesive, or the like, high curing performance required for a coating material is not required. The thermosetting resin composition according to the invention can reach a level that can be used as a coating material, and even a composition that does not reach such a level may be used in the field of an adhesive, a pressure-sensitive adhesive, or the like.

The invention relates to a cured film formed by three-dimensionally crosslinking the above-mentioned thermosetting resin composition.

Such a cured film has sufficient performance to be used as a coating material or an adhesive.

### Examples

Hereinafter, the invention will be described in more detail based on Examples. The invention is not limited to the following Examples. In the following descriptions, "part(s)" means "parts by weight".

### Example 1

Into a four-necked flask, 180 parts of succinic acid anhydride and 173 parts of methanol were charged, and succinic acid anhydride was dissolved at 60°C to 70°C. It was confirmed by NMR that the peak of succinic acid anhydride disappeared, and excess methanol was removed under a reduced pressure at 60°C or higher to synthesize monomethyl succinate.

190 parts of monomethyl succinate, 204.6 parts of glycidyl methacrylate, triethylbenzylammonium chloride, and a polymerization inhibitor were added and reacted at 90°C for 10 hours or longer to obtain a monomer 1.

A monomer mixed solution was prepared such that n-butyl methacrylate/monomer 1/4-hydroxybutyl acrylate/styrene = 35/30/25/10 (g/g). Next, 5 parts of PEROCTA O as an initiator was dissolved in aromatic hydrocarbon (T-SOL 100) to prepare an initiator solution.

Aromatic hydrocarbon (T-SOL 100) and propylene glycol monomethyl ether acetate were charged into a stirrable flask so as to be 50/50 (g/g), and the monomer mixed solution and the initiator solution were added dropwise while nitrogen was charged thereto. Polymerization was performed at 100°C for 4 hours or longer to obtain a polymer 1. Dioctyltin (NEOSTANN U-820, manufactured by Nitto Kasei Co., Ltd.) was added as a catalyst in an amount of 3 wt% based on the solid content, and the obtained thermosetting composition was subjected to a series of evaluations.

### Example 2

To the polymer 1 synthesized in Example 1, zinc acetylacetonate hydrate was added as a catalyst in an amount of 3 wt% based on the solid content, and the obtained thermosetting composition was subjected to a series of evaluations.

### Example 3

158.4 parts of monomethyl succinate synthesized in the same manner as in Example 1 and 247 parts of 3,4-epoxycyclohexylmethyl methacrylate (CYCLOMER-M100, manufactured by Daicel Corporation) are reacted in the same manner as described above to obtain a monomer 2.

A monomer mixed solution was prepared such that n-butyl methacrylate/monomer 2/4-hydroxybutyl acrylate/styrene = 35/30/25/10 (g/g). Next, 5 parts of PEROCTA O as an initiator was dissolved in aromatic hydrocarbon (T-SOL 100) to prepare an initiator solution.

Aromatic hydrocarbon (T-SOL 100) and propylene glycol monomethyl ether acetate were charged into a stirrable flask so as to be 50/50 (g/g), and the monomer mixed solution and the initiator solution were added dropwise while nitrogen was charged thereto. Polymerization was performed at 100°C for 4 hours or longer to obtain a polymer 2. Dioctyltin (NEOSTANN U-820, manufactured by Nitto Kasei Co., Ltd.) was added as a catalyst in an amount of 3 wt% based on the solid content, and the obtained thermosetting composition was subjected to a series of evaluations.

### Example 4

190 parts of monomethyl succinate synthesized in the same manner as in Example 1 and 201.5 parts of trimethylolpropane triglycidyl ether (EPOLITE 100MF, manufactured by Kyoeisha Chemical Co., Ltd.) were reacted in the same manner as described above to obtain a crosslinking agent 3.

A monomer mixed solution was prepared such that n-butyl methacrylate/2-hydroxybutyl methacrylate/styrene = 50/35/15 (g/g). Next, 5 parts of PEROCTA O as an initiator was dissolved in aromatic hydrocarbon (T-SOL 100) to prepare an initiator solution.

Aromatic hydrocarbon (T-SOL 100) and propylene glycol monomethyl ether acetate were charged into a stirrable flask so as to be 50/50 (g/g), and the monomer mixed solution and the initiator solution were added dropwise while nitrogen was charged thereto. Polymerization was performed at 100°C for 4 hours or longer to obtain a polymer 3. The polymer 3 and the crosslinking agent 3 were blended in the ratios shown in Table 2 below. Dioctyltin (NEOSTANN U-820, manufactured by Nitto Kasei Co., Ltd.) was added as a catalyst in an amount of 3 wt% based on the solid content, and the obtained thermosetting composition was subjected to a series of evaluations.

### Example 5

132 parts of monomethyl succinate synthesized in the same manner as in Example 1 and 136.4 parts of 3',4'-epoxycyclohexylmethyl 3,4-epoxycyclohexanecarboxylate (Celloxide 2010P, manufactured by Daicel Corporation) were reacted in the same manner as described above to obtain a crosslinking agent 4.

The polymer 3 and the crosslinking agent 4 were blended in the ratios shown in Table 2 below. Dioctyltin (NEOSTANN U-820, manufactured by Nitto Kasei Co., Ltd.) was added as a catalyst in an amount of 3 wt% based on the solid content, and the obtained thermosetting composition was subjected to a series of evaluations.

### Example 6

142.6 parts of monomethyl succinate synthesized in the same manner as in Example 1 and 247.1 parts of pentaerythritol triglycidyl ether (Denacol EX-411, manufactured by Nagase ChemteX Corporation) were reacted in the same manner as described above to obtain a crosslinking agent 5.

The polymer 3 and the crosslinking agent 5 were blended in the ratios shown in Table 2 below. Dioctyltin (NEOSTANN U-820, manufactured by Nitto Kasei Co., Ltd.) was added as a catalyst in an amount of 3 wt% based on the solid content, and the obtained thermosetting composition was subjected to a series of evaluations.

### Comparative Example 1

In the presence of potassium carbonate, 122 parts of acetoacetoxyethyl methacrylate and 98 parts of methyl acrylate were reacted at 40°C to 50°C for 6 hours or longer. Thereafter, neutralization and washing with water were performed to obtain a monomer 6.

A monomer mixed solution was prepared such that n-butyl methacrylate/monomer 6/4-hydroxybutyl acrylate/styrene = 35/30/25/10 (g/g). Next, 5 parts of PEROCTA O as an initiator was dissolved in aromatic hydrocarbon (T-SOL 100) to prepare an initiator solution.

Aromatic hydrocarbon (T-SOL 100) and propylene glycol monomethyl ether acetate were charged into a stirrable flask so as to be 50/50 (g/g), and the monomer mixed solution and the initiator solution were added dropwise while nitrogen was charged thereto. Polymerization was performed at 100°C for 4 hours or longer to obtain a polymer 4. Dioctyltin (NEOSTANN U-820, manufactured by Nitto Kasei Co., Ltd.) was added as a catalyst in an amount of 3 wt% based on the solid content, and the obtained thermosetting composition was subjected to a series of evaluations.

### Example 7

Into a four-necked flask, 140 parts of succinic acid anhydride and 315 parts of n-butanol were charged, and succinic acid anhydride was dissolved at 60°C to 70°C. It was confirmed by NMR that the peak of succinic acid anhydride disappeared, and excess n-butanol was removed under a reduced pressure at 80°C or higher to synthesize monobutyl succinate.

245 parts of monobutyl succinate, 160 parts of glycidyl methacrylate, triethylbenzylammonium chloride, and an inhibitor were added and reacted at 90°C for 10 hours or longer to obtain a monomer 7.

A monomer mixed solution was prepared such that n-butyl methacrylate/monomer 7/4-hydroxybutyl acrylate/styrene = 35/30/25/10 (g/g). Next, 5 parts of PEROCTA O as an initiator was dissolved in aromatic hydrocarbon (T-SOL 100) to prepare an initiator solution.

Aromatic hydrocarbon (T-SOL 100) and propylene glycol monomethyl ether acetate were charged into a stirrable flask so as to be 50/50 (g/g), and the monomer mixed solution and the initiator solution were added dropwise while nitrogen was charged thereto. Polymerization was performed at 100°C for 4 hours or longer to obtain a polymer 5. Dioctyltin (NEOSTANN U-820, manufactured by Nitto Kasei Co., Ltd.) was added as a catalyst in an amount of 3 wt% based on the solid content, and the obtained thermosetting composition was subjected to a series of evaluations.

### Examples 1 to 7 and Comparative Example 1

Each of the obtained thermosetting compositions was applied to a PET film (100 µm) at 400 µm (wet) using an applicator, and baked at 150°C for 30 minutes. The gel fraction was measured in the same manner. The physical properties in the table were measured by the following method.

In the table, the molecular weight means the weight average molecular weight, and is a value of the molecular weight in terms of polystyrene measured by gel permeation chromatography (GPC). GPC KF-804L (manufactured by Showa Denko K. K.) was used as a column, and tetrahydrofuran was used as a solvent.

### (Gel fraction)

The gel fraction was measured by dissolving the film obtained in Examples in an acetone reflux for 30 minutes using a Soxhlet, and measuring the remaining wt% of the film as the gel fraction.

The gel fraction of 0% to 40% was not acceptable for practical use and was evaluated as "D".

The gel fraction of 40% to 60% was evaluated as "C" because a certain degree of curing was observed.

The gel fraction of 60% to 80% was acceptable for practical use and was evaluated "B".

The gel fraction of 80% to 100% had excellent performance and was evaluated as "A".

### (Xylene rubbing)

Xylene rubbing was performed by rubbing the film obtained with a medical gauze impregnated with xylene 10 times, and observing the surface.

In the evaluation, a sample that was not acceptable for practical use was evaluated as "D", a sample that was acceptable for practical use was evaluated as "B", and a sample that had excellent performance was evaluated as "A".

### (Rigid pendulum tester)

Using a rigid pendulum tester (model number: RPT-3000W) manufactured by A&D Co., Ltd., after the temperature was increased to temperature (180°C) at a temperature increase rate of 10°C/min, the temperature was maintained, and the changes in cycle and logarithmic decrement at that time were determined. In particular, it was used to confirm the cured state of the coating film.
Pendulum: FRB-100
Film thickness (WET): 100 µm

### (Heat-resistance yellowing test)

A white paint (trade name: Amylac 4000, manufactured by Kansai Paint Co., Ltd.) was applied to a blister plate by spraying and was baked at 150°C for 30 minutes, and the value of b* at this time was used as a reference. (Tester: color difference meter CR DP400, manufactured by Konica Minolta, Inc.)

Each of the thermosetting compositions in Examples and Comparative Example was applied at 400 µm (wet) with a 100 µm applicator and baked at 180°C for 30 minutes. The value of b* at this time was calculated, and the difference was taken as Δb. Δb* was evaluated as A to D.
B: no yellowing was observed.
C: although yellowing was slightly observed, it was at a practical level.
D: yellowing was clearly observed and it was at an impractical level.

### (Weather resistance test)

A white paint (trade name: Amylac 4000, manufactured by Kansai Paint Co., Ltd.) was applied to a blister plate by spraying, was baked at 150°C for 30 minutes, and then was subjected to an irradiation test (irradiation amount: 100 mJ/cm² × 24 hours).

The value of b* at this time was used as a reference.

### (Tester: EYESUPER UVTESTER, manufactured by Iwasaki Electric Co., Ltd.)

Next, each of the thermosetting compositions in Examples and Comparative Example was applied onto a white base coat at 400 µm (wet) with a 100 µm applicator and baked at 150°C for 30 minutes to execute the test under the above conditions. b* at this time was measured, and the difference was defined as Δb. Δb* was evaluated as A to D.
B: no yellowing was observed.
C: although yellowing was slightly observed, it was at a practical level.
D: yellowing was clearly observed and it was at an impractical level.

**[Table 1]**

| | Example 1 | Example 2 | Example 3 | Comparative Example 1 | Example 7 |
|---|---|---|---|---|---|
| Polymer | 1 | 1 | 2 | 4 | 5 |
| Molecular weight | 10,000 | 10,000 | 7,300 | 9,600 | 10,500 |
| Baking temperature | 150°C | 150°C | 150°C | 150°C | 150°C |
| Baking time | 30 min | 30 min | 30 min | 30 min | 30 min |
| Gel fraction | A | A | A | A | A |
| Xylene rubbing | A | B | B | A | D |
| Heat-resistance yellowing test | B | C | B | D | B |
| Weather resistance test | B | C | B | D | B |
| Rigid pendulum test | FIG. 1 | FIG. 2 | FIG. 3 | FIG. 4 | FIG. 5 |

**[Table 2]**

| | Example 4 | Example 5 | Example 6 |
|---|---|---|---|
| Polymer solution 3 (solid content: 50%) Mw = 10,400 | 3 | 3 | 3 |
| Crosslinking agent 3 | 1.5 | | |
| Crosslinking agent 4 | | 1.5 | |
| Crosslinking agent 5 | | | 1.5 |
| NEOSTANN U-820 | 0.09 | 0.09 | 0.09 |
| Total | 4.50 | 4.50 | 4.50 |
| Gel fraction | A | A | A |
| Xylene rubbing | B | B | B |
| Heat-resistance yellowing test | B | B | B |
| Weather resistance test | B | B | B |
| Rigid pendulum test | FIG. 6 | FIG. 7 | FIG. 8 |

When the gel fraction was 40 or more, it was determined that a certain degree of curing reaction occurred, and it was clear that the composition had a function as a curable resin composition.

It was shown that in the thermosetting compositions obtained in Examples, a cured product having less yellowing and excellent transparency after the curing reaction by transesterification as compared with the related art can be obtained. In addition, since the result of xylene rubbing was good, it was suitable for use in a large number of applications including a coating material (in particular, a coating material for forming an outermost layer).

On the other hand, although the thermosetting composition in Comparative Example 1 had no problems in gel fraction and xylene rubbing, it was shown that yellowing clearly occurred based on results of thermal yellowing resistance and weather resistance. In Example 7, although there was no problem in weather resistance and gel fraction, the curing initiation temperature in the rigid pendulum test was low, and the evaluation of xylene rubbing was at a level that was difficult to put into practical use.

Thus, when the alkyl ester group is a methyl group, an excellent effect is exerted, and when the alkyl ester group is an n-butyl group, the curing performance is slightly inferior. However, even when the composition as in Example 7 is used, the composition can be suitably used in applications in which curing at a low temperature is not required and applications in which xylene rubbing is not required.

### Industrial Applicability

The thermosetting resin composition according to the invention can be used in applications of any known thermosetting resin composition such as a coating composition and an adhesive composition.

## Claims

1. An ester compound comprising:
in one molecule, at least one functional group represented by the following general formula (1) or (2): 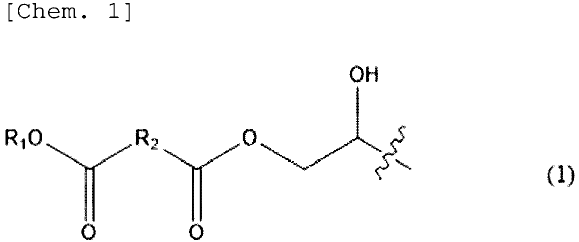 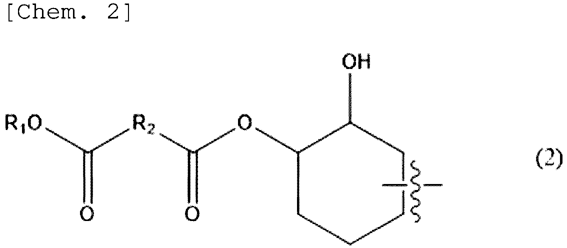
in both the general formulae (1) and (2), R₁ is an alkyl group having 50 or less carbon atoms, and
R₂ is an alkylene group having 50 or less carbon atoms that may contain an oxygen atom and a nitrogen atom as a portion thereof.

2. The ester compound according to claim 1, which is represented by the following general formula (3): 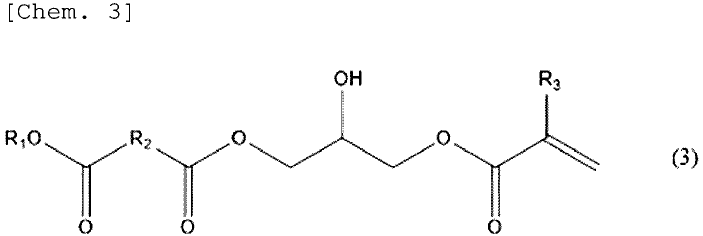
in the formula, R₁ is an alkyl group having 50 or less carbon atoms,
R₂ is an alkylene group having 50 or less carbon atoms that may contain an oxygen atom and a nitrogen atom, as a portion thereof, and
R₃ is hydrogen or a methyl group.

3. A method for producing the ester compound according to claim 1, comprising:
a step of reacting an acid anhydride, an alcohol, and an epoxy compound.

4. A polymer (A) comprising:
as at least a portion thereof, a structural unit derived from the ester compound according to any one of claims 1 to 3.

5. The polymer according to claim 4, which has a weight average molecular weight of 3,000 to 300,000.

6. The polymer (C) according to claim 4 or 5, further comprising:
as an essential structural unit, a structural unit derived from a hydroxy group-containing unsaturated monomer.

7. The polymer according to claim 6, wherein
the structural unit derived from a hydroxy group-containing unsaturated monomer includes, as at least a portion thereof, a structural unit derived from a monomer represented by the following general formula (4): 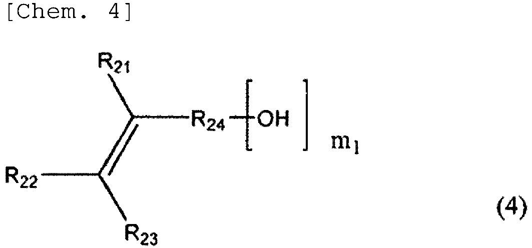
m₁: 1 to 10
in the formula, R₂₁, R₂₂, and R₂₃ are the same as or different from each other and each represent hydrogen, an alkyl group, a carboxyl group, an alkyl ester group, or a structure represented by the following R₂₄-[OH]m₁, and
R₂₄ is an aliphatic, alicyclic, or aromatic alkylene group having 3 or more and 50 or less atoms in a main chain, which may contain, in the main chain, one or two or more functional groups selected from the group consisting of an ester group, an ether group, an amide group, and urethane, or may have a side chain.

8. A thermosetting resin composition comprising:
a transesterification catalyst (F); and
a resin component (X) containing the functional group according to claim 1 and a hydroxy group.

9. The thermosetting resin composition according to claim 8, wherein
the resin component (X) is the polymer according to any one of claims 4 to 7.

10. A cured film, which is formed by three-dimensionally crosslinking the thermosetting resin composition according to claim 8 or 9.
